# EUROPEAN PATENT SPECIFICATION

(11) **EP 1 899 461 B1**
(45) Date of publication and mention of the grant of the patent: **08.12.2010**
(21) Application number: 06818202.1
(22) Date of filing: 09.06.2006
(51) Int. Cl.: C12N 9/12

(54) **NEW PHOSPHORYLATION SITE OF MITOGEN-ACTIVATED PROTEIN KINASES, MODIFIED PROTEINS AND APPLICATIONS**
NEUER PHOSPHORYLIERUNGSSTANDORT FÜR MITOGENAKTIVIERTE PROTEINKINASEN UND MODIFIZIERTE PROTEINE SOWIE ANWENDUNGEN DAVON
NOUVEAU SITE DE PHOSPHORYLATION DE PROTEINES KINASES ACTIVEES PAR DES AGENTS MITOGENES, PROTEINES MODIFIEES ET APPLICATIONS

(30) Priority: 10.06.2005 ES 200501404
(43) Date of publication of application: 19.03.2008
(73) Proprietor: Universidad Autonoma de Madrid, 28049 Madrid (ES)
(72) Inventor: MURGA MONTESINOS, Cristina, Ctra. De Colmenar Viejo Km 15 (ES); MAYOR MENÉNDEZ, Federico, Ctra. De Colmenar Viejo Km 15 (ES); JURADO PUEYO, María, Ctra. De Colmenar Viejo Km 15 (ES); CAMPO MUELAS, Pedro Manuel, Ctra. De Colmenar Viejo Km 15 (ES); PEREGRIN PEDRIQUE, Sandra, 03550 Sant Joan d'Alacant (ES)
(74) Representative: ABG Patentes, S.L.
(86) International application number: PCT/EP2006/005542
(87) International publication number: WO 2007/028430

(56) References cited:
- WO-A-95/07922
- WO-A2-03/056340
- PEARSON G ET AL: "Mitogen-activated protein (MAP) kinase pathways: Regulation and physiological functions" ENDOCRINE REVIEWS, BALTIMORE, MD, US, vol. 22, no. 2, April 2001 (2001-04), pages 153-183, XP002235969 cited in the application

## Description

### FIELD OF THE INVENTION

The invention relates to a new phosphorylation site of mitogen-activated protein kinases (MAPK), to the modified MAPKs in said phosphorylation site and to their applications, wherein said MAPK protein is a p38 protein.

### BACKGROUND OF THE INVENTION

### Mitogen-Activated Protein Kinases (MAPK)

The term MAPK includes three kinase cascades, ERK, JNK and p38 and their respective isoforms [Pearson G., et al., 2001, "Mitogen-activated protein (MAP) kinase pathways: Regulation and Physiological Functions", Endocrine Reviews 22(2):153-183]. The cellular effects mediated by these kinases are numerous and cover the whole life cycle of a cell: growth, division, differentiation, motility, osmotic responses, response to stress, inflammation, cancer, etc.

The detection of a certain extracellular stimulus is transmitted to a first kinase, called MAPKKK the targets of which are the serines and threonines of another kinase, MAPKK. This phosphorylation determines MAPKK activation by phosphorylating serine and threonine residues in a limited T-Xaa-Y triad or three-amino acid motif (in which T is threonine, Y is tyrosine and Xaa is a residue of an amino acid such as, for example, aspartic acid, glutamic acid, glutamine, glycine or proline) called activation segment, carrying the final target of this trimodular cascade. MAPK is the effector kinase in charge of phosphorylating several substrates, such as transcription factors, other kinases, structural elements etc., in serine and threonine.

### P38

Protein p38 MAPK, or p38, is an enzyme belonging to the family of serine/threonine kinases and it plays an important role in the cellular response to external stress signals, such as ultraviolet light, osmotic shock, heat, etc. For this reason, this protein is also known as stress-activated protein kinase or SAPK. p38 carries out its regulating role by controlling gene expression through phosphorylation and activation of transcription factors, of other kinases and also by regulating the stability of important messenger RNA.

There are four p38 isoforms differing in their distribution in different tissues and in their sensitivity to different p38 inhibitors, although the most studied and therefore, the best known one is the alpha isoform, the activation of which has been observed in many cellular types (both in hematopoietic and non-hematopoietic tissue) after treatment with a suitable stimulus. However, in spite of these differences, all p38 isoforms have an activation domain formed by 12 amino acids which contains the activation segment Thr-Gly-Tyr phosphorylated by MKK6/MKK3, enzymes of the kinase family found upstream p38 in the cell signaling cascade.

p38 is an essential regulator of cell functions that mediates the production of cytokines and other molecules responsible for the development of inflammatory processes and takes part in different physiological situations induced by cellular stress, as in the case of some cardiopathies and inflammatory phenomena, and in cell cycle control.

Throughout several years, the involvement of p38 in the development or evolution of different diseases has been analyzed. The importance of p38 activation in establishing and developing cardiac failure has already been established. A constitutive activation after aortic constriction has been observed in experimental models in mice and in a model in hypertensive rats with high salt diets. In humans, p38 is activated in hearts affected by heart failure following advanced coronary disease. The regulation of p38 activation seems to be essential in the development of heart pathology since several groups have observed a reduction in p38 activity in end-stage heart failure in human and rat myocardium. By using p38 MAPK inhibitors, the involvement of p38 MAPK in other pathologies differing from heart diseases, such as inflammatory, pulmonary or neuronal diseases have also been described in the state of the art. All these pathologies are characterized in that they have active p38, i.e., with the phosphorylated Thr180 and Tyr182 residues.

On the other hand, it has been observed in cells derived from cancer patients that both chemotherapy and radiotherapy produce a p38 activation that seems to be responsible for the signal inducing the death of tumor cells.

The most wide-spread strategy in the treatment of the different diseases characterized by the presence of active p38 consists of the pharmacological inhibition of its activity or of the inhibition of its activation, as mentioned in WO 2005/032551, WO 2004/021988, EP 1534282 or CA2497448, combined with other therapeutic agents.

### GRKs

G protein-coupled receptors (GPCR) mediate the actions of different messengers carrying out an essential role in cardiovascular system or immune system functions. In addition to interacting with heterotrimeric G proteins, activated GPCRs interact with G protein-coupled receptor kinases (GRKs) and with modulating proteins called arrestins. Based on structural similarities, the 7 members of the GRK family (GRK-7) have been classified in 4 subfamilies, GRK1, GRK2/3, GRK4/5/6 and GRK7, where GRK2/3 and GRK5/6 have a ubiquitous distribution in the organism. However, the mechanism that alters GPCR signaling and contributes to the triggering and/or progression of these pathologies is not known.

These proteins play an essential role in the rapid modulation of the intracellular functionality and dynamics of receptors after activation by ligands in addition to allowing the recruitment and regulation of other cell proteins, starting new signaling routes, therefore, they are both essential modulators and components of GPCR-mediated signal transduction. The levels and functionality of several GRKs are altered in pathological situations such as congestive heart failure, cardiac hypertrophy, hypertension or inflammatory processes such as rheumatoid arthritis.

On the other hand, the important role of SAPKs in the development of cardiomyopathies and heart failure is known and it is also known that the selective GPCR activation promotes chronic activation of these kinases in the heart muscle, this step being essential in the development of heart failure from ventricular hypertrophy.

### BRIEF DESCRIPTION OF THE FIGURES

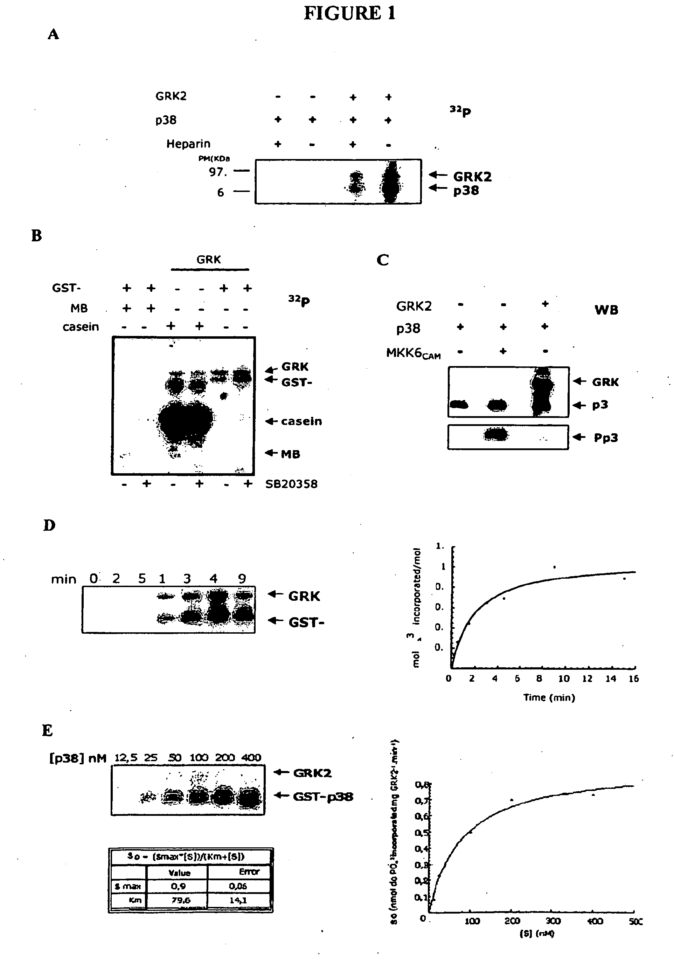

**Figure 1** shows that GRK2 directly phosphorylates p38 *in vitro.* Figure 1A shows that p38 is phosphorylated by GRK2. Recombinant GRK2 purified by the Baculovirus system (50 nM) was incubated with 50 nM of recombinant p38 (GST-p38) in p38 phosphorylation buffer (25 mM Hepes, pH 7.5, 10 mM magnesium acetate, 50 µM ATP, 2000-3000 cpm/pmol [γ-³²P]ATP) in a final volume of 40 µl for 30 minutes at 30°C, with or without heparin (50 ng/µl) for the purpose of inhibiting GRK2 activity. Kinase autophosphorylation controls were carried out in the same conditions. The reaction was stopped by adding SDS loading buffer, the proteins were separated in an 8% SDS-polyacrylamide gel and developed by autoradiography. Figure 1B shows that GRK2 directly phosphorylates p38 and does not promote its autophosphorylation. The phosphorylation reactions were carried out as indicated in relation to Figure 1A. Heparin and SB203580 compounds, GRK2 and p38 inhibitors respectively, were used at ten times the IC₅₀ concentration so as to assure the complete inhibition of the respective kinases, that is, at 1.5 µM for heparin and at 0.5 µM for SB203580. The substrates used were MBP (14 µg per point) for p38 and casein (7.5 µg per point). The reaction was stopped by adding SDS loading buffer, the proteins were separated in a 10% SDS-polyacrylamide gel and detected by autoradiography. Figure **1C** shows that GRK2 does not phosphorylate p38 in its activation segment. The phosphorylation reactions were started in the absence of radioactive ATP in the conditions previously described. 40 ng of MKK6_{CAM} (MKK6/MKK3, Upstate) were used to phosphorylate GST-p38 *in vitro.* The antibodies used for the detection after electrophoretic separation recognize, in the case of larger proteins, GRK2 and GST-p38 (anti-PF2 antibody, polyclonal serum, generated in the inventors' laboratory, with antibodies against the GST-PF2 fusion protein, in which PF2 is a C-terminal fragment of GRK2).The phosphospecific antibody of T180/Y182 used to detect the p38 activation state is of Cell Signaling. Figure **ID** shows the dependence on time of p38 phosphorylation by GRK2. The phosphorylation was left to be carried out in the same conditions as those described previously, except for the incubation time, which as indicated, ranged from 0 to 90 minutes. The mobility of both proteins (GRK2 79.6 kDa; GST-p38 68 kDa) is shown by arrows. The data obtained for the excised bands of the gels after quantifying by Cerenkov are shown on the right-hand side. Figure **IE** shows the phosphorylation kinetic parameters. In view of the data of section D, 15 minutes of reaction were considered to be the time period for determining phosphorylation kinetic constants in initial speed conditions. The phosphorylation reactions were carried out as has already been described keeping the GRK2 concentration fixed (25nM) and changing the p38 concentration from 12.5 to 400 nm. The reaction was stopped by adding SDS loading buffer, the proteins were resolved in an 8% SDS-polyacrylamide gel, detected by autoradiography and quantified by Cerenkov. The graph was made with the Kaleidagraph program, provide with an algorithm capable of deducing kinetic parameters: the Michaelis-Menten constant (Km=79.56 nM) and maximum speed (Smax=0.9 nmol of PO₄³⁻ incorporated per mg GRK2⁻¹minute⁻¹).

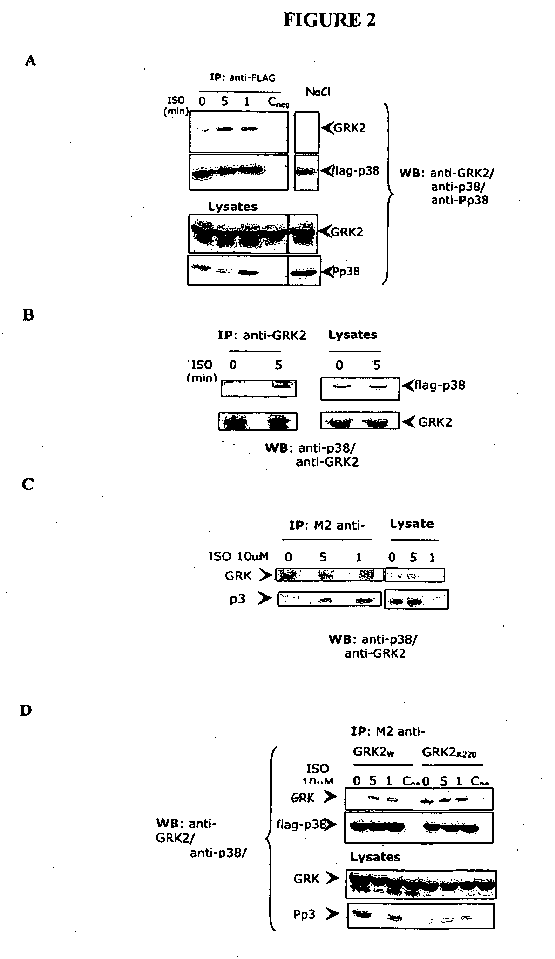

**Figure 2** shows that GRK2 and p38 are dependently associated to the β₂-adrenergic (β₂-AR) receptor stimulation. Figure **2A****:** HEK 293 cells were transiently transfected with pCDNA3-GRK2, pBC12BI-β2-AR and pCDNA3-Flag-p38α (1µg of each DNA per p60) vectors. 48 hours after the transfection and after a 2 hour serum starvation, the cells were stimulated with the adrenergic agonist isoproterenol (ISO, 10 µM) for 5 or 10 minutes or they were not stimulated (0 min ISO). Likewise, cells without overexpressed β₂-AR receptor were stimulated with 0.5 M NaCl for 15 minutes. The cells were solubilized in a buffer suitable for immunoprecipitation with the M2 anti-Flag antibody bound to agarose and after taking aliquots of these lysates (10%) for overexpression control (lysate panels) the immune complexes were incubated overnight at 4°C. The immunoprecipitated proteins (Ip anti-Flag) were resuspended in an electrophoresis breaking buffer and the bound proteins were resolved in a 10% SDS-polyacrylamide gel. After electrotransference, the proteins were detected with anti-p38 and with the antibody against GRK2, anti-PF2 (upper and lower panel, respectively). The negative immunoprecipitation controls (Cneg) in the absence of Flag-p38a and of stimulation with 10 µM isoproterenol are also included in the figure. In the lysate panels, GRK2, in the upper part, and the p38 activation state, in the lower part, were detected by means of the anti-phospho-p38 antibody (see Table 1) Figure **2B****:** In approaches identical to those described in 2A, the overexpressed proteins were immunoprecipitated with the antibody against GRK2 anti-PF2 and the co-immunoprecipitation of the latter was detected with anti-p38 in each stimulation point (0 and 5 minutes of isoproterenol, left upper panel). The Flag-p38 expression controls (upper part) and GRK2 expression controls (lower part) and the total immunoprecipitated GRK2 (lower left panel) are shown in adjacent panels. The association between both proteins is again detected, stimulated 5 minutes after exposure to 10 µM isoproterenol. Figure **2C****:** This time, only the vectors encoding β₂-AR and Flag-p38α were introduced in HEK293 cells for the purpose of assuring that endogenous GRK2 was co-immunoprecipitated in a total amount lower than p38 (0.5 µg of DNA). The maximum association between Flag-p38α and GRK2 is produced 5 minutes after treatment with 10 µM isoproterenol (see upper and lower left panels). The right-hand panels confirm GRK2 expression (upper part, anti-PF2) and p38 expression (lower part, anti-p38) in the cellular lysates. Figure **2D****:** In experiments identical to those described in section A, the ability of the catalytically inactive GRK2-K220R mutant to co-immunoprecipitate with Flag-p38 was tested. The two upper panels show, respectively, the co-immunoprecipitation of the two GRK2 isoforms and the total immunoprecipitated p38. The two lysate panels show GRK2 and GRK2-K220R overexpression (upper part, anti-PF2) as well as the Flag-p38 activation state in each point (lower part, anti-phospho-p38). As described in A, non-nspecific immunoprecipitation controls (Cneg) of the M2 anti-Flag-agarose antibody were carried out.

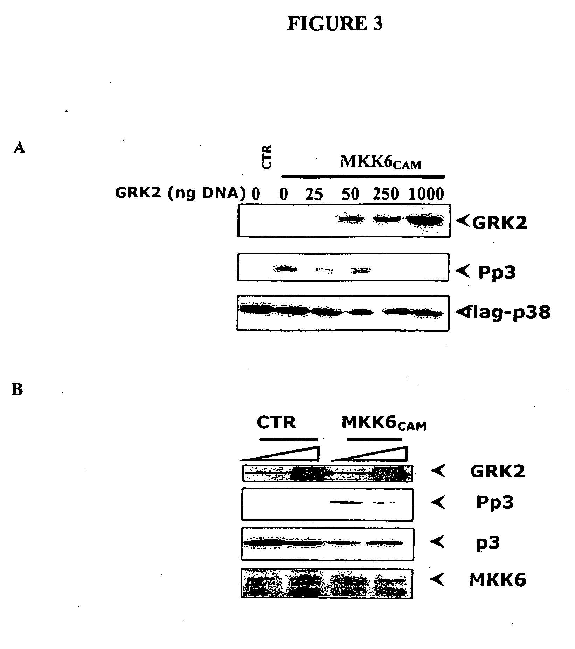

**Figure 3** shows how GRK2 is capable of reducing p38 activation by MKK6_{CAM}. Figure **3A****:** HEK 293 cells, normally seeded in multiwell plates with 6 or 12 wells (M6 or M12), were transiently transfected with pCDNA3-Flag-p38α, pCDNA3-MKK6_{CAM}, and with increasing amounts of the pCDNA3-GRK2 vector. In this experiment, M6 plates were used and the DNA amounts introduced were: 100 ng of Flag-p38, 100 ng of MKK6_{CAM}, and 0 to 1 µg of GRK2. In all points, the total amount of DNA was completed with pCEFL-EGFP and with empty pCDNA3 instead of pCDNA3-MKK6_{CAM}, in the case of the control (CTRL). After 48 hours, the cells were harvested by lysing them in M2 buffer. The upper panel shows the overexpressed GRK2 dose. The p38 activation state was evaluated with anti-phospho-p38, of Cell Signaling, and after giving off the antibodies of the first immunodetection, it was detected with total anti-p38, of the same company. Note that very slightly exposed autoradiographies are show in order to avoid low resolution saturations of the development. Figure **3B****:** Two mass-cultures of EBNA 293 cells stably transfected with pCDNA3-GRK2 and therefore overexpressing two different GRK2 levels were transfected with Flag-p38 and MKK6_{CAM}. The Flag-p38 activation state was determined in the same way as in A. Furthermore, the correct MKK6_{CAM} expression was assured with the anti-MKK6/SKK3 antibody of Upstate Biotechnology.

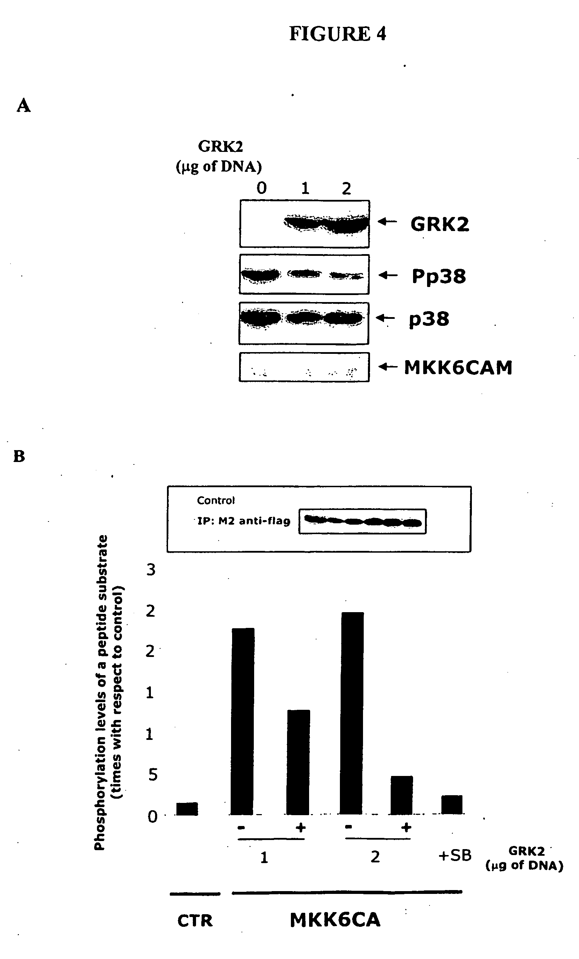

In **Figure 4****,** the experiments show that GRK2 reduces the catalytic activity of p38. Figure **4A****:** HEK 293 cells, seeded in p60, were transiently transfected with 0.5 µg of pCDNA3-Flag-p38a, 0.5 µg of pCDNA3-MKK6_{CAM} (or 0.5 µg of empty pCDNA3 in the control, CTRL), and with 1 or 2 µg of the pCDNA3-GRK2 (+) vector, carrying out each point in duplicate. The controls were carried out in a parallel way, substituting the pCDNA3-GRK2 µg with pCEFL-EGFP (-). The total DNA amount was completed with empty pCDNA3. After 48 hours, the cells were lysed, lysate aliquots were taken and Flag-p38 was immunoprecipitated from them. The panels in A show the GRK2 (anti-PF2) and the MKK6_{CAM} (anti-MKK6) overexpression and the Flag-p38 (anti-phospho-p38) activation. Figure **4B****:** The immunoprecipitates were washed three times with 15 ml of M2 buffer and two times with 15 ml of phosphorylation buffer without ATP (15mM NaF, 25 mM Hepes pH 7.5 and 10mM magnesium acetate). In the last washing step, the agarose-immune complexes were resuspended in 1 ml of buffer and 10% of each point was separated to control the immunoprecipitation of Flag-p38 (panel inserted in the graph of section B). Kinase-assays were carried out with immunoprecipitated Flag-p38, using APRTPGGRR peptide (initially provided by CalbioChem and subsequently synthesized by the Servicio de Quimica de Proteínas *(Protein Chemistry Service)* of the CBMSO) as a substrate. The reactions were carried out in a final volume of 25 µl, in a phosphorylation buffer formed by 25 mM Hepes pH 7.5; 10 mM magnesium acetate, 15 mM NaF, 50 µM ATP and 500-1000 cpm/pmol [γ-³²P] ATP and 2 mM of the peptide substrate. When (+SB) is specified, SB203580 was added to the *in vitro* reaction at a final concentration of 0.5 µM. The phosphorylation was left to take place for 30 minutes at 30°C, after which it was stopped by adding 15 µl of 30% TCA. The proteins were precipitated by centrifugation (25 000 xg, 15 minutes, 4°C) and the supernatant containing the phosphorylated peptide was recovered from each reaction. Square (1 cm x 1 cm) Whatman P81 paper cut-outs were impregnated with the peptide in solution. They were left to dry, were abundantly washed with 75 mM phosphoric acid and the radioactivity incorporated by the adsorbed peptide was quantified by Cerenkov. The activity levels are referred to peptide substrate phosphorylation by Flag-38 in the absence of MKK6_{CAM} (CTRL).

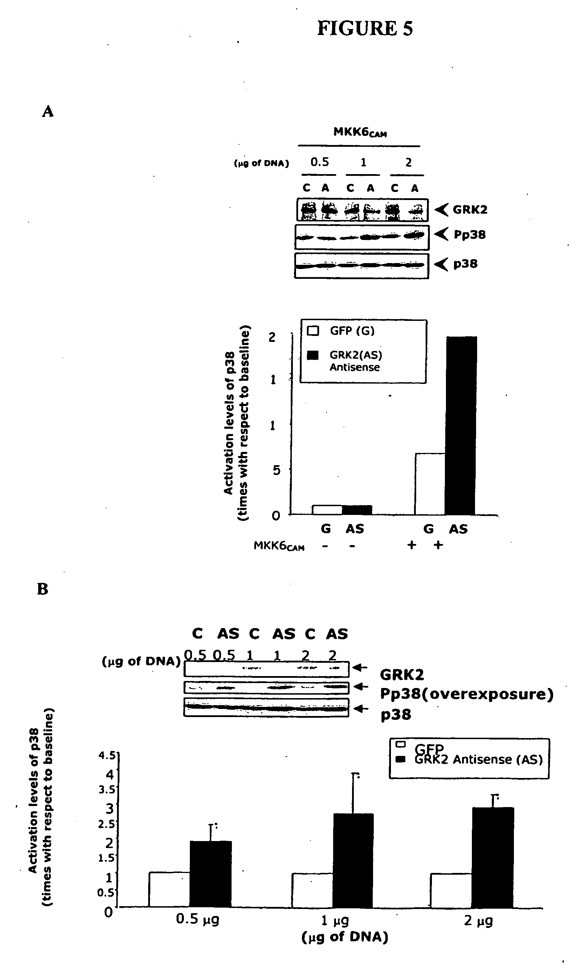

**Figure 5** shows that the reduction of GRK2 levels allows greater p38 activation by MKK6_{CAM} *in situ.* Figure **5A****;** HEK 293 cells, seeded in M6 plates, were transiently transfected with 150 ng of pCDNA3-Flag-p38α, 50 ng of pCDNA3-MKK6_{CAM}, and with increasing amounts of the pCEFL-GRK2 antisense (AS) vector: 0.5 µg to 2 µg or the same amounts of pCEFL-EGFP as a control (C). In all the points, the total DNA amount was completed with the empty pCEFL vector. The cells were lysated and the immunodetection of the total amount of GRK2 (upper panel, anti-PF2), p38 activation (intermediate panel, anti-phospho-p38) and total p38 (lower panel, anti-p38N) was carried out in each point. The data from the 2 µg (of GFP and Antisense) point of a representative experiment are shown in the graph. The p38 activation levels in each of the two conditions refer to their respective controls without MKK6_{CAM}. Figure **5B****:** The basal p38 activation is modulated by the amount of GRK2. M6 plates of HEK 293 cells were transiently transfected with 100 ng of pCDNA3-Flag-p38a, and with 0.5 µg to 2 µg of pCEFL-GRK2 antisense or of pCEFL-EGFP. The total DNA amount in each point was completed with pCEFL. The total amount of GRK2 (anti-PF2, upper panel), the p38 activation state (intermediate panel, anti-phospho-p38, autoradiography overexposed during the chemiluminescent developing) and total p38 (lower panel, anti-p38) were evaluated by electrotransference and immunodetection in these conditions. The mean values (± SEM) of data from three independent studies are shown in the graph of the lower part of the Figure in duplicate. The two-sided Student's t-test statistic with *p<0.005 was applied to determine significance.

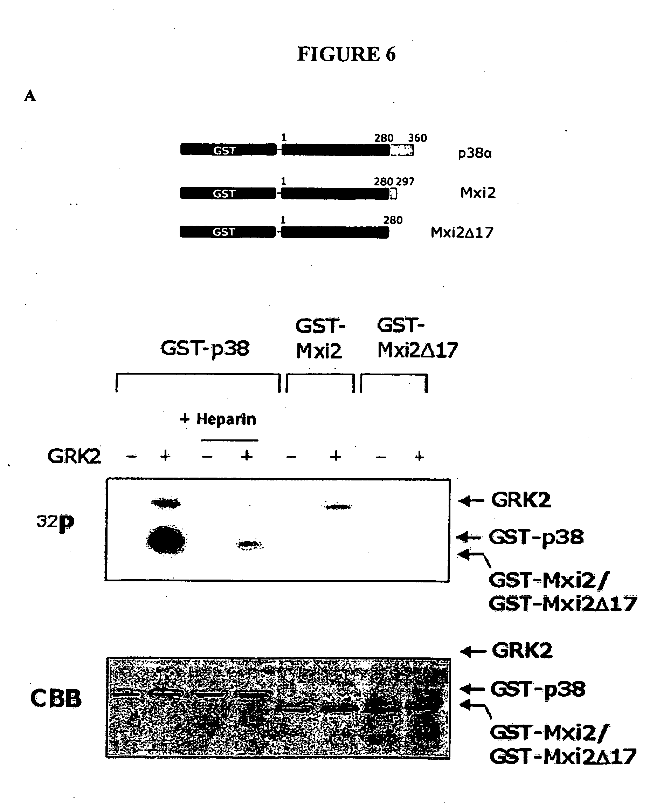

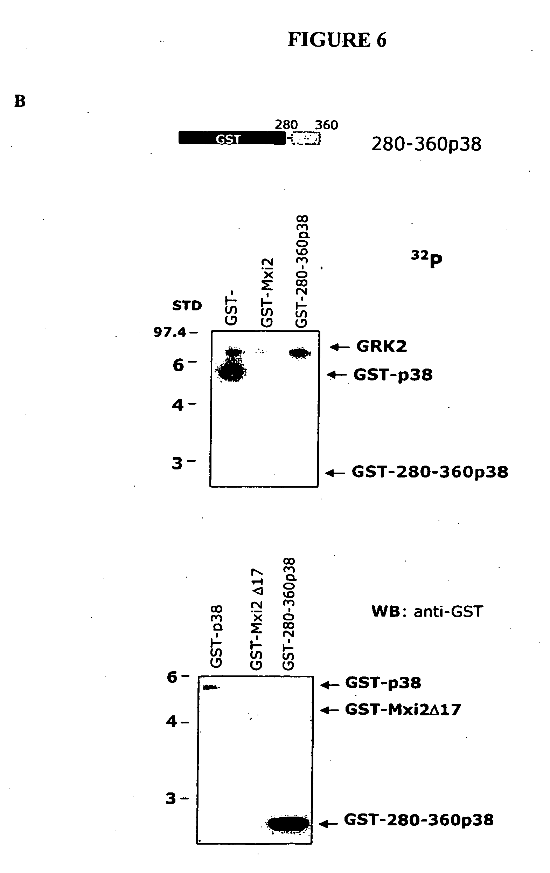

**Figure 6** shows that only p38, with its integral structural determinants, is a GRK2 substrate. Figure **6A****:** Proteins fused to GST (black rectangle in the protein schemes) were purified from bacteria transformed with the plasmids pGEX2T- p38a, pGEX4T-Mxi2 and pGEX4T-Mxi2Δ17: p38α, Mxi2 and Mxi2Δ17. The Mxil isoforms used as C-terminal truncated mutants for trying to limit the GRK2 phosphorylation site. Recombinant GRK2 (200 nM) was incubated with 0.5 µg of each fusion protein, in phosphorylation buffer (25 mM Hepes pH 7.5, 10 mM magnesium acetate, 50 µM ATP, 2000-3000 cpm/pmol [γ-³²P]ATP) for 30 minutes to 30°C. Heparin (150 nM) was included as a specific GRK2 inhibitor. The reactions were stopped by adding SDS breaking buffer. The samples were resolved in 8% SDS-PAGE. For the purpose of assuring the inclusion of identical amounts of protein, they were first visualized in the gel by Coomassie Brilliant Blue (CBB) staining. Then the gel was dried and the radioactivity incorporated to the proteins (³²P) was detected. Figure **6B****:** The truncated GST-280-360p38 protein, corresponding to the last 80 p38α amino acids, was generated by means of the Invitrogen Gateway system. Phosphorylation assays were carried out with Mxi2Δ17 as a p38 N-terminal, 280-360 p38 as C-terminal and with whole p38α in the same conditions as described in section A. The phosphorylation was detected by autoradiography (³²P) and controls of the amount of protein used in the assays were carried out concomitantly by Western blot with the anti-GST antibody. The presence of the GST-280-360p38 protein, which is not phosphorylated, is clearly observed. STD, mobility of standard molecular weight proteins.

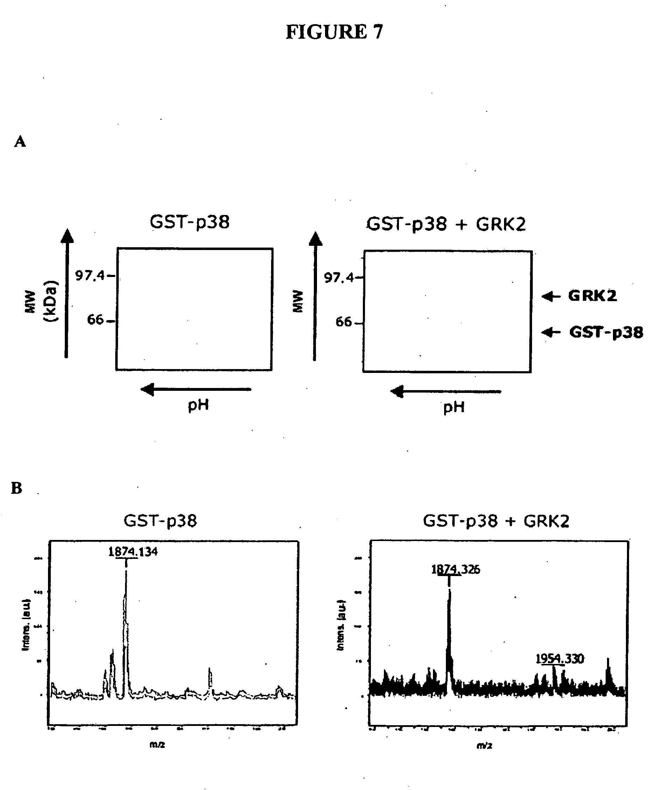

**Figure 7** shows that GRK2 phosphorylates p38 in a single residue. Figure **7A****:** p38 phosphorylation by GRK2 was analyzed in two-dimensional electrophoresis and it was left to take place in the same conditions as described previously. The reaction was stopped by adding an isoelectrofocusing loading buffer. The first electrophoretic dimension had an ampholyte gradient from pH 3 to 10.The second dimension was resolved by means of an 8% polyacrylamide gel. The mobility of both proteins (GRK2 79.6 kDa; GST-p38 68 kDa) is indicated by arrows. Figure **7B****:** The samples from a non-radioactive phosphorylation assay were run in a gel intended for proteomic sequencing. The gel was stained in order to visualize the bands, and the one corresponding to GST-p38 was excised from the gel and subjected to tryptic digestion. The resulting peptides were analyzed by MALDI-TOF. An enlargement of the area of the obtained mass spectrum is shown in which the phosphorylated peptide (mass corresponding to 1,945.330), present only in the sample from phosphorylation by GRK2, was detected.

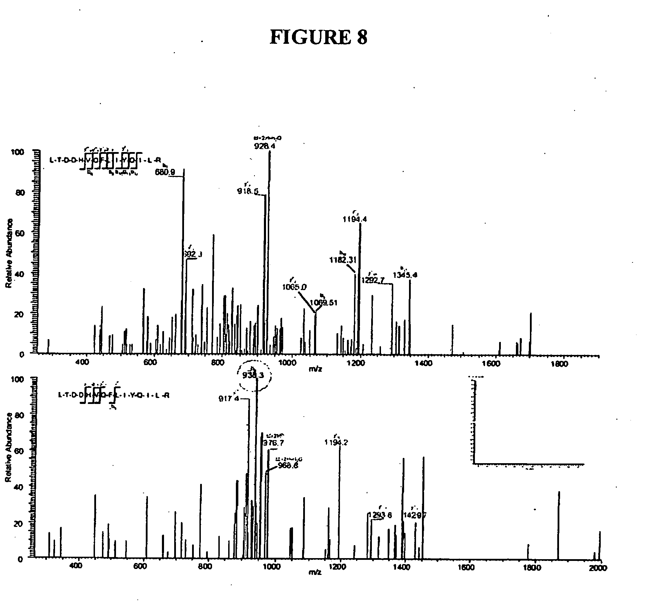

**Figure 8** shows that GRK2 phosphorylates p38 in a single residue. With the data obtained in MALDI-TOF (Broker Autoflex model), a tryptic peptide, a candidate for carrying phosphorylation, was identified: LTDDHVQFLIYQILR. In order to verify these indications, the sample was resolved by HPLC-ESI-IT (Thermo-Finnigan Deca-XP model), working in SIM (single ion monitoring) mode so that the analyzer only fragmented the candidate peptide. A series (called bₙ or y"ₙ, according to the course of the fragmentation) was assigned to the recognizable peptide masses. A mass corresponding to the Δb₈ series (in the orange disk) indicating phosphorylation in the threonine present in the analyzed peptide is seen in the lower spectrum, from the phosphorylated sample. Likewise, the total mass of the peptide (inside the yellow oval) was identified in both cases. The miniature window inserted in the right-hand side of the Figure shows the information about differential elution in the HPLC column of the same peptide from the phosphorylated sample and the control. Given its greater hydrophilia, the phosphorylated peptide is eluted a few tenths of a second before.

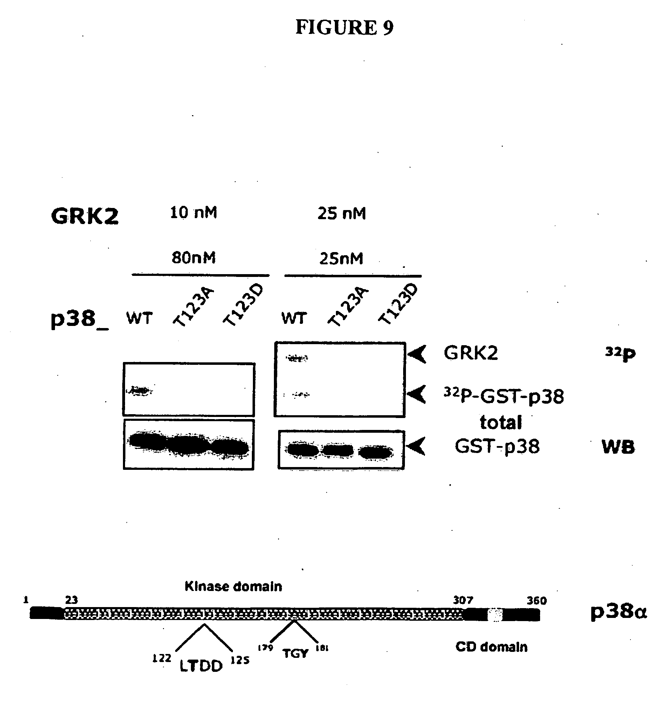

**Figure 9** shows that p38 T123A and T123D mutants are not phosphorylated by GRK2 *in vitro.* Figure **9A:** p38T123A and p38T123D proteins, fused to GST, were made by directed mutagenesis in the prokaryotic expression plasmids pGEX2T. The ability of the recombinant GST-p38T123A and GST-p38T123D proteins to be GRK2 substrates in comparison to the wild GST-p38WT protein and at the final concentrations indicated in the Figure was assayed. The upper panels represent the obtained phosphorylation (³²P) and the lower panels show, by anti-p38N WB, the amount of recombinant protein used in the assays. Figure **9B:** Representative scheme of p38α in which the functional characteristics are highlighted, such as the kinase domain extension, the location within the same of the TGY activation segment and of the LTDD sequence hypothetically regulated by GRK2. The CD motif, initially involved in regulating substrate and activator association, is also highlighted.

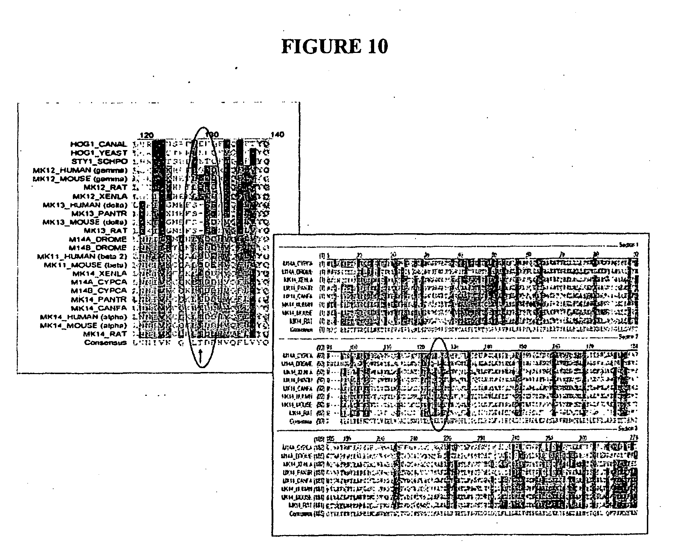

**Figure 10** shows that threonine 123 is a highly conserved residue, located on the outer surface of the docking groove for p38 activators and substrates. The multiple alignments that appear were made by the ClustalX program (http://www-igbmc.u-strasbg.fr/BioInfo/ClustalX/) by Dr. Perdiguero. The dashes introduce gaps for adjusting the alignment. The identical residues among all isoforms are highlighted in red writing on a yellow background, blue and green backgrounds indicate conservative substitutions, according to large or small distribution, respectively, between the aligned proteins. Non-conserved amino acids are left on white background. The consensus is indicated at the foot of each multiple alignment and by means of red ellipses, the threonine 123 area is highlighted, pointed out additionally in the sequence comparison in the lower part with an arrow.

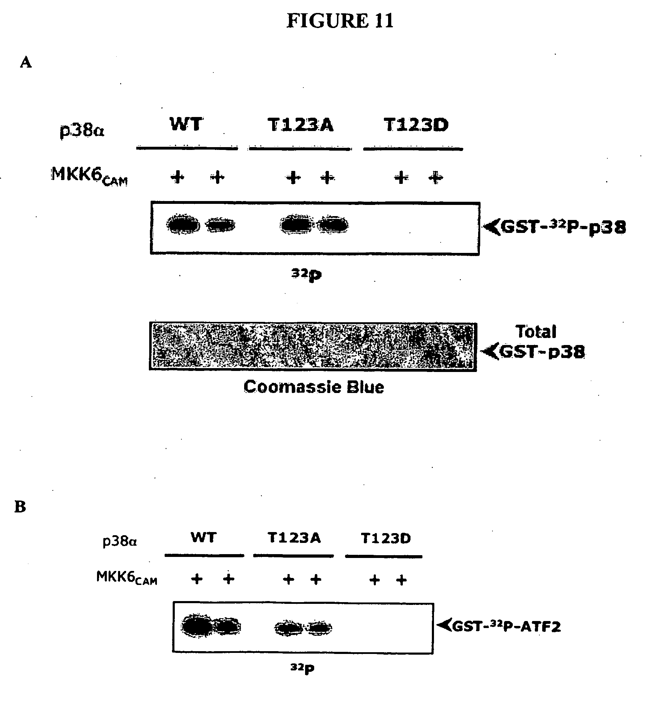

**Figure 11** shows that the p38T123D mutant is not activated by MKK6_{CAM} and lacks kinase activity on ATF2 *in vitro.* Figure **11A****:** Phosphorylation assays (25 mM Hepes pH 7.5; 10 mM magnesium acetate, 15 mM NaF, ATP 50 µM and 1000-2000 cpm/pmol [γ-³²P]ATP) were carried out *in vitro* with the fusion proteins GST-p38WT, GST-p38T123A and GST-p38T123D (150 nM) as recombinant MKK6_{CAM} (40 ng) phosphorylation substrates (Upstate Biotechnology). The reactions were carried out at 30°C for 30 minutes and the proteins were separarated in 8% SDS-PAGE gels. The amount of p38 in each point was visualized by Coomassie Blue staining. Then the radioactivity in each p38 isoform was determined. The autoradiography (³²P) shows a representative experiment of three independent assays, carried out in duplicate. Figure **11B****:** In these same assays, the ability of MKK6_{CAM}-activated GST-p38WT, GST-p38T123A and GST-p38T123D to phosphorylate 2 µg of GST-ATF2 is evaluated. The representative autoradiography (³²P) of the total GST-ATF2 control (Coomassie Blue) is attached.

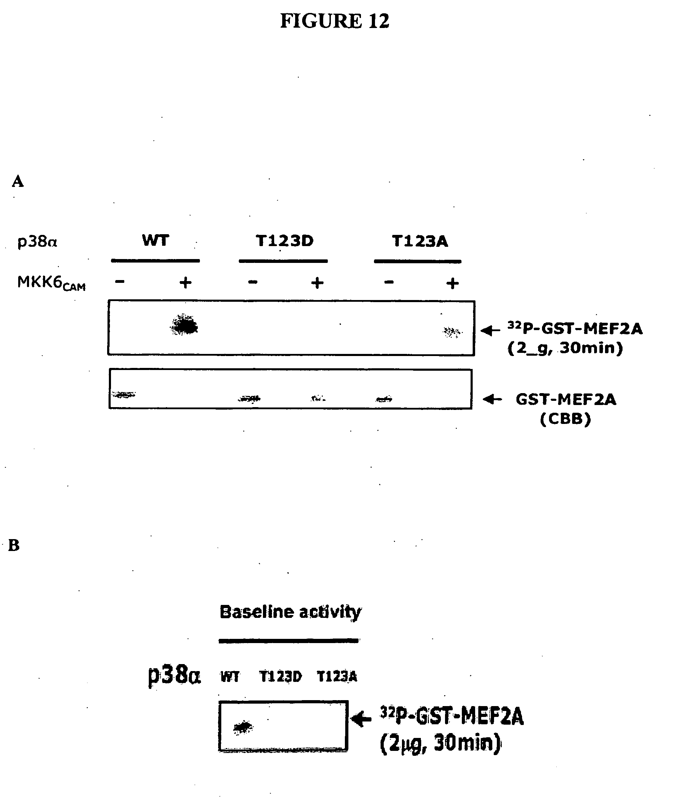

**Figure 12** shows that p38 threonine 123 is necessary for its correct catalytic activity on the MEF2A substrate. Figure **12A****:** *In vitro* phosphorylations are carried out with the fusion proteins indicated in the Figure, in the presence (+) or absence (-) of recombinant MKK6_{CAM} (40 ng) and 2 µg of GST-MEF2A. The assay was left to take place for 30 minutes. The proteins were separated in 8% SDS-PAGE, stained with CBB and the incorporated radioactivity (³²P) was detected. Panels of a representative experiment of the three assays carried out independently are provided. Figure **12B****:** The baseline activity of the three p38 fusion proteins in the absence of the MKK6_{CAM} activator was evaluated in the previously described conditions. A representative autoradiography after a long exposure is shown.

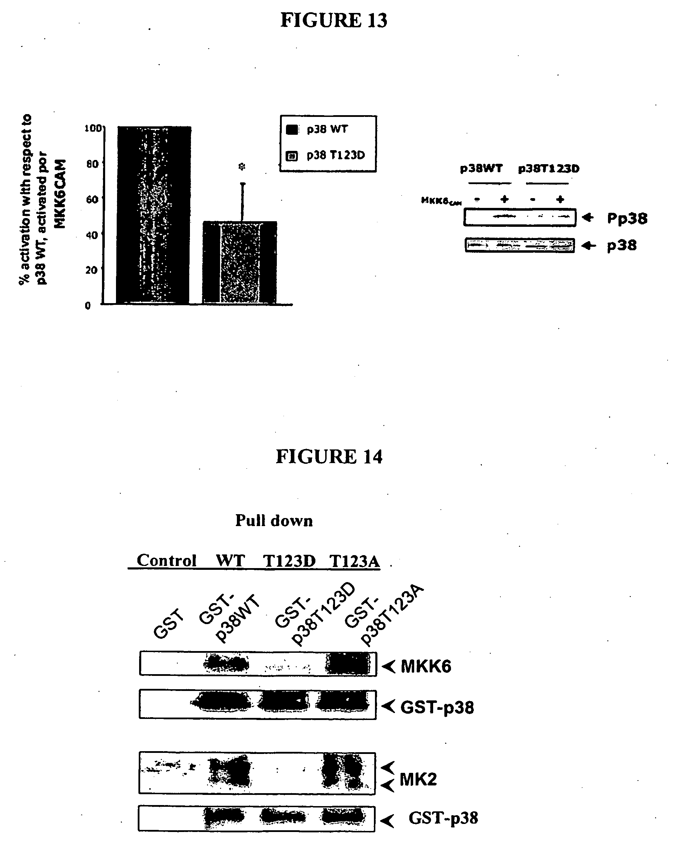

**Figure 13** shows that the p38 T123D mutant is less activated by MKK6_{CAM} *in situ* than wild p38. In overexpression experiments analogous to those described previously, HEK293 cells, seeded in M6 and always in duplicate, were transfected with: 150 ng of pCDNA3-Flag-p38α WT, or pCDNA3-Flag-p38α T123D and 50 ng of pCDNA3-MKK6_{CAM}, (or empty pCDNA3). p38 activation with the anti-phospho-p38 antibody was evaluated. For each of the two isoforms, the activation by MKK6_{CAM} was in reference to the baseline situation, obtaining a certain activation level. 100% activation was established as the p38α WT activation in each experiment. Data (± SEM) of three independent experiments are shown. A two-sided Student's-t test was carried out and *p<0.0001 was obtained. The right-hand side panels (Western blot) illustrate one of the experiments.
**Figure 14** shows that the p38 T123D mutant does not bind substrates, and it does not associate to or become phosphorylated by MKK6. Purified GST-p38 and its T123 mutants (300 nM) were incubated in the presence of purified MKK6 (3 nM) or His-tagged MAPKAPK2 (MK2, 20 nM) in a pull down assay with GST as a negative control. Sedimented proteins were developed by Western Blot using anti-MKK6, anti-Histidine (for His-MK2) or anti-GST (for total amounts of GST-p38s) antibodies. In all panels, results are representative of three independent experiments performed in duplicate.

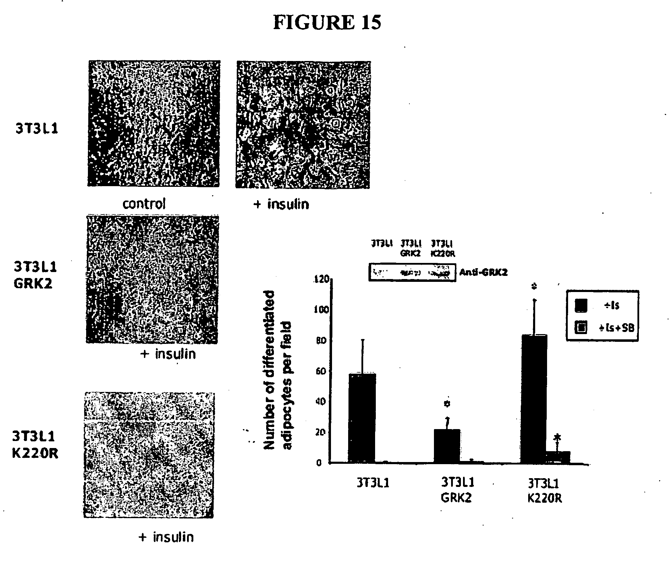

**Figure 15** shows how GRK2 reduces the p38-dependent differentiation of fibroblasts to adipocytes. 3T3L1 lines stably transfected with pCDNA3-GRK2 and pCDNA3-GRK2K220R were made. GRK2 (anti-GRK2) expression controls obtained in cell lines, compared to 3T3L1 levels, are included. The cells were subjected to a standard differentiation treatment and on day 15 of the latter, the cells were fixed with formalin and stained with Oil Red, a lipophilic coloring which binds to the fats accumulated by the adipocytes. The cells with adipocytic phenotype (in red) were counted under microscope in a total of 25 fields. Representative photographs of two independent experiments are shown in duplicate. A photo of the 3T3L1 cells, free of stimulation with insulin during the differentiation process (control), is also included. The graph reflects the adipocyte count (± SEM) in these experiments. A two-sided Student's-t test was carried out for each stable line with respect to the 3T3L1 cells and *p<0.0001 was obtained. p38 dependence controls were carried out in parallel by means of pharmacological treatment with 10 µM of SB203580.

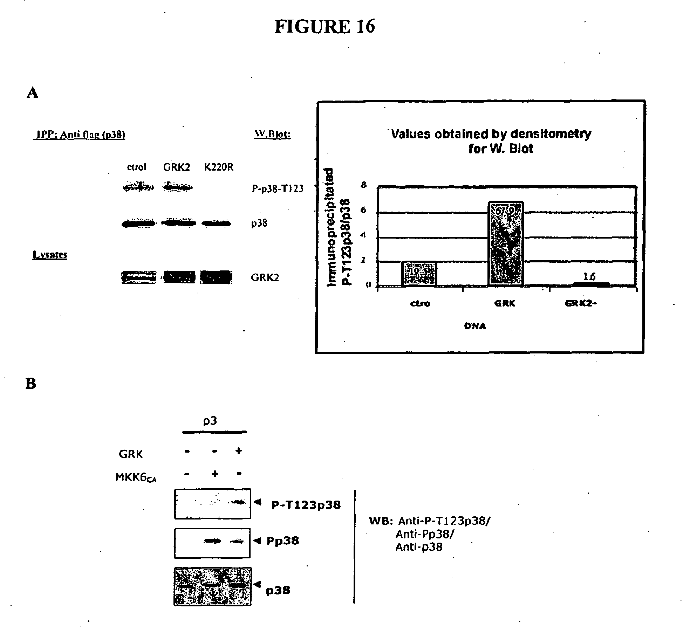

**Figure 16** represents recognition by the anti-phospho-Thr123 antibody of p38 immunoprecipitated from human cells in culture and the *in vitro* phosphorylation of p38 by GRK2. Figure **16A****:** HEK293 cells were transfected with expression vectors for mouse Flag-p38alpha and GRK2 or its inactive mutant (GRK2-K220R). The cells were cultured for 16 hours without serum before lysing. Flag-p38 was immunoprecipitated (IPP) by means of an anti flag (M2) antibody and, after electrophoresis and transference, the phosphorylated protein was detected at Thr123 by means of Western Blot (WB) with a specific antibody purified in affinity columns (P-p38-T123, dilution 1:200). The same membrane was developed with total anti-p38 (p38) and another membrane corresponding to the proteins contained in the cell extracts before immunoprecipitation (lysates) with an anti-GRK2 436-689(GRK2). Figure **16B****:** S GST-p38 (50 nM) was incubated alone, with purified GRK2 (150 nM) or with 40 ng of constitutively active MKK6_{CAM} in phosphorylation buffer in the presence of non-radioactively labeled ATP for 30 minutes at 30°C. The upper panel was developed with a polyclonal antibody against Thr123 of p38 in phosphorylated form. The intermediate panel was incubated with the phosphospecific antibody of the Thr180/Tyr182 residues of p38 (Anti-Pp38) and an antibody against total p38 was used in the lower development.

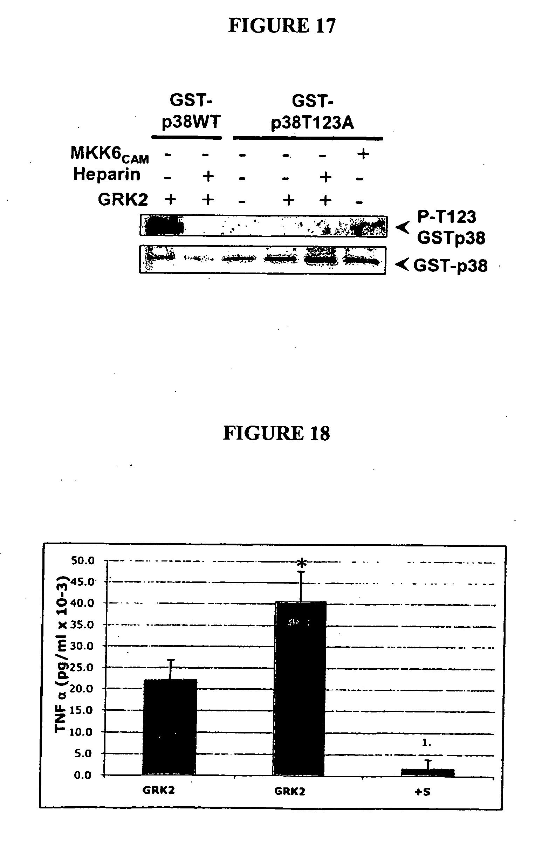

**Figure 17** shows that an anti-phospho antibody recognizes p38 phosphorylated by GRK2 at threonine 123 since the T123A mutant of p38 is not recognized in this conditions. GST-p38wt and GST-p38T123A (70 nM) were incubated alone with purified GRK2 (25 nM) or with recombinant MKK6CAM (2 ng); where indicated, heparin (100 ng/µl), a specific GRK2 inhibitor, was added. Phosphorylation of GSTp38 proteins was analyzed by Western Blot with anti-phosphoT123 and total GST-p38 with anti-GST. Data are representative of 3 independent experiments.
**Figure 18** shows the p38 activation levels and TNF secretion levels in response to lipopolysaccharide of macrophages extracted from partially GRK2-deficient mice. Peritoneal microphages from wild type C57BL/6 (+/+) or GRK2 hemizygous (+/-) mice. After subjecting them to the absence of stimulation for 2 hours, bacterial lipopolysaccharide (LPS: 0.5 µg/ml) was added to the culture medium for 16 hours. The production of inflammatory cytokines (TNFα) was quantified by means of a commercial ELISA kit (Amersham Biotrak). To confirm the dependence of this process on p38 activity, SB203580 (30 µM) was used for 30 minutes before adding LPS. The means ±SD of 10 mice processed in 4 independent experiments are shown. The data corresponds to TNFα secretion derived from 106 cells per well of M24. * p<0.005 (according to the Student's t-test).

### DETAILED DESCRIPTION OF THE INVENTION

In an aspect, the invention relates to an MAPK protein, wherein said MAPK protein is a p38 protein, occasionally identified in this description as "MAPK protein of the invention", selected from:
a) an MAPK protein comprising a phosphorylated residue in a phosphorylation site that is different from the phosphorylation site or sites present in the activation segment of said MAPK protein, or a fragment of said protein comprising said phosphorylated residue, wherein
   - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
   - the phosphorylation at said different phosphorylation site prevents the activation of said MAPK protein and also its activity towards its substrates; and
b) an MAPK protein comprising a negative charge or a bulky residue in a phosphorylation site, or at the area surrounding said phosphorylation site, that is different from the phosphorylation site or sites present in the activation segment of said MAPK protein, or a fragment of said protein comprising said phosphorylated residue, wherein
   - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
   - the introduction of a negative charge or a bulky residue at said phosphorylation site, or at the area surrounding said phosphorylation site, prevents the activation of said MAPK protein and also its activity towards its substrates.

As it is used herein, the term "positionally equivalent" refers to the position of an amino acid of a MAPK protein which, by multiple alignment of amino acid sequences of MAPK proteins, corresponds to Thr123 of mouse p38, α isoform.

The term "MAPK protein" includes the ERK, JNK and p38 protein kinases, as well as their respective isoforms, of any species. Information on said kinases and their functions as well as on their cellular effects can be found in the review carried out by Pearson et al. [Pearson G., et al., 2001, "Mitogen-activated protein (MAP) kinase pathways: Regulation and Physiological Functions", Endocrine Reviews 22(2):153-183]. Information on the amino acid sequences of said MAPK proteins can be found in suitable databases known by the persons skilled in the art (e.g., Swissprot, NCBI, etc.). MAPK kinases are widely distributed among the different species and their primary structure is widely conserved among the different members of the different families (ERK, JNK and p38).

MAPK proteins are characterized, *inter alia,* by the existence of an activation segment comprising at least one residue susceptible of being phosphorylated by the suitable kinase.

In a particular embodiment, said activation segment comprises the amino acid triad of formula (I)

Thr-Xaa-Tyr (I)

where
Thr is threonine,
Tyr is tyrosine, and
Xaa is the residue of an amino acid.

In a particular embodiment, Xaa is the residue of an amino acid selected from aspartic acid, glutamic acid, glutamine, glycine and proline.

In the particular case of mammal p38, α isoform, the activation segment comprises the amino acid triad of formula (I) in positions 180-182 of its amino acid sequence.

In another particular embodiment, said activation segment comprises the amino acid triad of formula (II)

Ser-Glu-Gly (II)

where
Ser is serine,
Glu is glutamic acid, and
Gly is glycine.

The MAPK protein of the invention is a p38. p38 kinase is widely distributed among the different species and its primary structure is widely conserved (Figure 10). In a particular embodiment, said p38 is mammal p38, for example of a human, rodent, etc., in any of its isoforms (α, β, γ, or δ). In a specific embodiment, said p38 is the α isoform of mouse p38 *(Mus musculus)* the amino acid sequence of which is shown in SEQ ID NO: 1 (GenBank, Access number P4781 1).

The inventors have surprisingly found that the phosphorylation of an MAPK protein, wherein said MAPK protein is a p38 protein, in a site susceptible of phosphorylation that is different from the phosphorylation site or sites present in the activation segment of said MAPK protein is able to inhibit the activation of said MAPK protein, which activation, as is known, occurs through phosphorylation of the residues susceptible of phosphorylation (e.g., threonine or tyrosine) present in said activation segment, for example specifically in said amino acid triad to which reference has previously been made.

In fact, studies carried out by the inventors have clearly shown that the phosphorylation of a threonine residue in position 123 (Thr123) of mouse p38, α isoform, prevents the phosphorylation of the threonine and tyrosine residues present in positions 180 and 182, respectively, of the amino acid triad of formula (I) present in the activation segment of said p38. As a result, p38 cannot be activated, and therefore it cannot carry out its function in the signal transduction cascade, which may be particularly useful in the treatment of those diseases in which the activation of said MAPK in the cell signaling cascade is involved.

The skilled person in the art will understand that, not only phosphorylation at said new phosphorylation site, but also the introduction of a negative charge or a bulky residue at said new phosphorylation site, or at the area surrounding said site, may also cause the effect of preventing the activation of an MAPK protein, wherein said MAPK protein is a p38 protein, and its activity towards its substrates.

Therefore, the invention teaches the existence of a new phosphorylation site present in an MAPK protein, wherein said MAPK protein is a p38 protein, wherein said phosphorylation site is different from the phosphorylation site or sites present in the activation segment of said MAPK protein, such as Thr123 of mouse p38, α isoform, or a residue of a positionally equivalent amino acid susceptible of phosphorylation in another MAPK protein as it is defined by multiple alignment of amino acid sequences, and it furthermore has the particularity that once it is phosphorylated, it is able to inhibit activation of said MAPK protein.

The specific location of said different phosphorylation site may vary depending on the MAPK protein in question (ERK, JNK or p38), the isoform and the animal species, although neither its function of preventing the activation of the MAPK protein in question after its phosphorylation (or after introducing a negative charge or a bulky residue in said phosphorylation site or at the area surrounding said site) nor its positional equivalence or correspondence will vary. Any MAPK protein, wherein said MAPK protein is a p38 protein, containing said phosphorylation site with the previously mentioned positional and functionality characteristics is included within the scope of the present invention. Therefore, the MAPK protein of the invention not only includes the phosphorylated mouse p38 protein, α isoform, at Thr123 but also its orthologous proteins, i.e. proteins which, coming from a common ancestral gene, carry out the same function in the different species, as well as their isoforms, irregardless of if the phosphorylation site is at said location (Thr123) or at another different position and the phosphorylated amino acid is an amino acid that is different from threonine.

Additionally, the MAPK protein of the invention also includes a modified MAPK protein, wherein said MAPK protein is a p38 protein, having a negative charge or a bulky residue at the new phosphorylation site or at the area surrounding said site, e.g., a modified mouse p38 protein, α isoform, containing a negative charge or a bulky residue at Thr123, or at the area surrounding said site, but also its orthologous proteins, as well as their isoforms, irregardless of if the negative charge or bulky residue is at said location (Thr123) or at another different position and the modified amino acid is an amino acid that is different from threonine. Illustrative, non limitative examples of negative charges which may be introduced into said new phosphorylation site, or at the area surrounding said site, according to the invention, will be evident for the skilled person in the art, for example, any molecule or compound capable of providing a negative charge, e.g., a peptide carrying a phosphate group, said peptide being capable of binding to said phosphorylation site, or to the surrounding area thereof, and mimicking the effect of the phosphorylation at that site. Illustrative, non limitative examples of bulky residues which may be introduced into said new phosphorylation site, or at the area surrounding said site, according to the invention, will be evident for the skilled person in the art, for example, any molecule, e.g., peptide or a low molecular weight compound, capable of binding to said phosphorylation site, or to the surrounding area thereof, and mimicking the effect of the phosphorylation at that site; although the inventors do not want to be joined by any theory, it is believed that said bulky residue may cause a conformational change in the MAPK protein which prevents the activation of said MAPK protein and also its activity towards its substrates. As it is used herein, the expression "at the area surrounding the (new) phosphorylation site" means a region around the phosphorylation site wherein a modification introduced therein by means of a negative charge or a bulky residue prevents the activation of said MAPK protein and also its activity towards its substrates. Suitable assays for determining the effect of preventing or inhibiting the activation of an MAPK protein and its activity towards its substrates can be found in the accompanying Example; thus, said information can be used by the skilled person in the art in order to identify said "area surrounding the phosphorylation site".

In a particular embodiment, the MAPK protein of the invention is a fragment of an MAPK protein, wherein said MAPK protein is a p38 protein, comprising a phosphorylated residue in a phosphorylation site that is different from the phosphorylation site or sites present in the activation segment of said MAPK protein, wherein, as previously mentioned, said different phosphorylation site is Thr123 of mouse p38, α isoform, or a residue of a positionally equivalent amino acid susceptible of phosphorylation in another MAPK protein as it is defined by multiple alignment of amino acid sequences, and the phosphorylation at said different phosphorylation site prevents the activation of said MAPK protein.

The length of said fragment may vary within a broad interval, for example between 3 and 30 amino acid residues, typically between 5 and 25 amino acid residues, preferably between 10 and 20 amino acid residues. Nevertheless, if desired said fragment may contain a larger number of amino acid residues. Advantageously, said fragment comprises an epitope of an MAPK protein of the invention. In a particular embodiment, said fragment comprises SEQ ID NO: 2 corresponding to the epitope QKLpTDDHVQFLIY, where "pT" represents the phosphorylated Thr123 residue and the remaining letter indicate the annotation of the amino acids based on a single-letter code of mouse p38 kinase, α isoform. Said epitope is highly conserved throughout evolution, therefore said SEQ ID NO: 2 can be considered to be a consensus sequence of said epitope among the orthologous proteins of p38 of different species.

In another particular embodiment, the MAPK protein of the invention is a fragment of an MAPK protein, wherein said MAPK protein is a p38 protein, comprising a negative charge or a bulky residue in a phosphorylation site (or at the area surrounding said site) that is different from the phosphorylation site or sites present in the activation segment of said MAPK protein, wherein, as previously mentioned, said different phosphorylation site is Thr123 of mouse p38, a isoform, or a residue of a positionally equivalent amino acid susceptible of phosphorylation in another MAPK protein as it is defined by multiple alignment of amino acid sequences, and said modification (negative charge or bulky residue) at said different phosphorylation site prevents the activation of said MAPK protein. The length of said fragment may vary within a broad interval, for example between 3 and 30 amino acid residues, typically between 5 and 25 amino acid residues, preferably between 10 and 20 amino acid residues. Nevertheless, if desired said fragment may contain a larger number of amino acid residues. Advantageously, said fragment comprises an epitope of an MAPK protein of the invention.

A number of pathologies are known in which the activation of MAPK is involved. By way of a non-limiting illustrative example, the relationship existing between different diseases and active p38, i.e. phosphorylated in the phosphorylation residues present in the activation segment, for example in the Thr180 and Tyr182 residues of mammal (mouse) p38, α isoform, is known. Therefore, the fact that the activation of MAPK, wherein said MAPK protein is a p38 protein, can be prevented (preventing phosphorylation in the phosphorylation sites present in the activation segment of MAPK) by phosphorylation or introduction of a negative charge or a bulky residue at said new phosphorylation site or at the area surrounding the new phosphorylation site of said MAPKs identified in the present invention (e.g. phosphorylation in Thr123 prevents phosphorylation in the Thr180 and Tyr182 residues of mammal (mouse) p38, α isoform), and also the fact that phosphorylation or introduction of a negative charge or a bulky residue in Thr123 or at the area surrounding Thr123 can prevent the docking and activity of p38 towards its substrates have important biological implications that are useful, *inter alia,* in the diagnosis of a pathology mediated by an active MAPK, or for determining the risk or predisposition of a subject of developing said pathology, or for evaluating or monitoring the effect of a therapy administered to a subject who has said pathology, or for analyzing the stage or severity and/or the evolution of said pathology, as well as in the identification of potentially useful compounds for the treatment of said pathology. The MAPK protein of the invention may play a significant role in this sense.

The term "subject" includes any member of an animal species, including human beings; by way of illustration, said subject can be a mammal, such as a human being, a domestic animal, a rodent, etc., preferably a man or woman of any age and race.

The expression "pathology mediated by an active MAPK" as it is used herein includes any pathology in which an active MAPK, i.e. phosphorylated in the phosphorylation residues present in the activation segment, is involved or plays a role. Illustrative, non-limiting examples of said pathology mediated by an active MAPK include cancer and cardiac, infectious, neuronal, pulmonary and inflammatory diseases. Illustrative non-limiting examples of said diseases include myocardial infarction, hypertrophia, hypertension, myocarditis, angioplasty-induced lesions, myocardial dysfunctions, viral or bacterial infections, neuronal death or death of other cell types, Alzheimer's disease, psoriasis, rheumatoid arthritis, autoimmune neuritis, Crohn's disease, cancer (carcinomas, leukemias, lymphomas, sarcomas, etc.), formation of clots, response to chemotherapeutic and radiotherapeutic agents, response to ischemia/reperfusion, etc.

Therefore, it is herein disclosed that the MAPK protein of the invention is a protein useful as a diagnostic marker or as a marker of the predisposition of a subject of developing a pathology mediated by an active MAPK, for example cancer or a cardiac, infectious, nervous, neuronal, pulmonary or inflammatory disease. Given that the presence of an active MAPK is associated with the abovementioned pathologies mediated by active MAPKs, the identification of the MAPK protein of the invention would be indicative of a lower risk or predisposition of developing said pathology because the phosphorylation in the phosphorylation site identified in the present invention, or the introduction of a negative charge or a bulky residue in said phosphorylation site or at the area surrounding said site, would prevent the activation of said MAPK. In a particular embodiment, said MAPK protein of the invention is a phosphorylated mammal p38 protein in a phosphorylation site that is different from the phosphorylation site or sites present in the activation segment of said mammal p38, for example a phosphorylated mammal p38 in Thr123, or a fragment of said protein comprising said phosphorylated residue.

It is also disclosed an *in vitro* method for analyzing the risk or predisposition of a subject of developing a pathology mediated by an active MAPK, comprising:
a) detecting and/or quantifying the level of an MAPK protein of the invention in a biological sample from said subject; and
b) comparing said level with the level of a control sample,
wherein a reduction in said level with respect to the level of the control sample is indicative of the risk of the subject of developing said pathology mediated by an active MAPK.

Virtually any biological sample from the subject to be studied can be used, for example blood, serum, plasma, tissue, etc. Said sample can be obtained by conventional methods. The control sample is a sample from subjects that do not suffer said pathology mediated by an active MAPK and includes reference or baseline values.

The detection and/or quantification of the level (concentration) of said MAPK protein of the invention can be determined by conventional methods known by the persons skilled in the art, for example by means of immunochemical methods (see below).

It is also disclosed that the MAPK protein of the invention can also be used for evaluating or monitoring the effect of a therapy administered to a subject who has said pathology mediated by an active MAPK, for example, cancer or a cardiac, infectious, nervous, neuronal, pulmonary or inflammatory disease. In this sense, a treatment preventing the activation of MAPK, for example by phosphorylating in the phosphorylation site identified in the present invention, or by introducing a negative charge or a bulky residue in said phosphorylation site or at the area surrounding said site, would allow analyzing the effect of a therapy administered to a subject who has said pathology and, if it is not effective, modifying the treatment or designing a customized therapy. In a particular embodiment, said MAPK protein of the invention is a phosphorylated mammal p38 protein in a phosphorylation site that is different from the phosphorylation site or sites present in the activation segment of said mammal p38, for example, a phosphorylated mammal p38 in Thr123, or a fragment of said protein comprising said phosphorylated residue.

It is also disclosed that the MAPK protein of the invention can also be used for analyzing the stage or severity and/or the evolution of said pathology mediated by an active MAPK, for example, cancer or a cardiac, infectious, nervous, neuronal, pulmonary or inflammatory disease. In this sense, the identification of an MAPK protein of the invention would be indicative of a better evolution of this type of pathologies. In a particular embodiment, said MAPK protein of the invention is a phosphorylated mammal p38 protein in a phosphorylation site that is different from the phosphorylation site or sites present in the activation segment of said mammal p38, for example, a phosphorylated mammal p38 in Thr123, or a fragment of said protein comprising said phosphorylated residue.

It is also disclosed an *in vitro* method for evaluating or monitoring the effect of a therapy administered to a subject who has said pathology mediated by an active MAPK, or for analyzing the stage or severity and/or the evolution of said pathology mediated by an active MAPK, comprising
a) detecting and/or quantifying the level of an MAPK protein of the invention in a biological sample from said subject; and
b) comparing said level with the level of a control sample from the same subject.

The comparison between both levels will be indicative of the efficacy of the treatment and/or of the evolution of the pathology. To that end, in this case the control sample is a sample from the subject before administering the treatment or in periods subsequent to the administration of the treatment for analyzing the efficacy thereof and the evolution of the pathology.

The detection and/or quantification of the level (concentration) of said MAPK protein of the invention can be determined by conventional methods known by persons skilled in the art, for example by means of immunochemical methods (see below).

The MAPK protein of the invention can also be used to identify potentially useful compounds for the treatment of said pathology mediated by an active MAPK, wherein said MAPK protein is a p38 protein, for example, cancer or a cardiac, infectious, nervous, neuronal, pulmonary or inflammatory disease. In this sense, compounds preventing the activation of said MAPK can be used in the treatment of said cardiac, infectious, nervous, neuronal, pulmonary or inflammatory diseases; compounds dephosphorylating the MAPK of the invention can also be used for the treatment of cancer because the activation of said MAPKs after subjecting a subject to chemotherapy or radiotherapy produces the signal that induces the death of tumor cells. In a particular embodiment, said MAPK protein of the invention is a phosphorylated mammal p38 protein in a phosphorylation site that is different from the phosphorylation site or sites present in the activation segment of said mammal p38, for example, a phosphorylated mammal p38 in Thr123, or a fragment of said protein comprising said phosphorylated residue, or a p38 having a compound bound to Thr 123 or to the area surrounding Thr123 area and imitates the presence of the phosphate negative charge or bulky residue in Thr123 or at the area surrounding the Thr123 residue.

In a particular embodiment, the invention provides an *in vitro* method for identifying a potentially useful compound for the treatment of pathologies mediated by MAPK proteins, comprising:
a) placing the candidate compound in contact with an MAPK protein,
b) detecting the phosphorylation of said MAPK protein in a phosphorylation site different from the phosphorylation site or sites present in the activation segment of said MAPK protein, and
c) analyzing if said phosphorylation site (i) is Thr123 of mouse p38, α isoform, in the event that the MAPK protein used was said protein, or a residue of a positionally equivalent amino acid susceptible of phosphorylation in another MAPK protein as it is defined by multiple alignment of amino acid sequences, and if (ii) the phosphorylation in said different phosphorylation site prevents the activation of said MAPK protein;
   or alternatively,

i) placing the candidate compound (e.g., a compound capable of phosphorylating said MAPK protein or a compound that mimics the effect of said phosphorylation) in contact with an MAPK protein;
ii) detecting the phosphorylation of said MAPK protein in a phosphorylation site of the activation segment of said MAPK protein to measure the effect of the candidate compound on the activation of the MAPK, or detecting the effect of mimicking said phosphorylation on said MAPK protein to measure the effect of the candidate compound on the activation of the MAPK;
iii) analyzing the activity of the said MAPK protein in the presence of the candidate compound towards its substrates to test the possible inhibition of the docking and/or activity of the MAPK protein to its substrates in the presence of a competing compound; and
iv) analyzing if said phosphorylation site (i) at Thr123 of mouse p38, α isoform, (in the event that the MAPK protein used was said protein) is affected by the candidate compound and if (ii) the phosphorylation in said phosphorylation site prevents the activation of said MAPK protein.

The candidate compound may be, in a particular embodiment, a compound capable of phosphorylating an MAPK protein (e.g., a kinase, etc.) or a compound that mimics the effect of said phosphorylation) in contact with an MAPK protein. The competing compound may be, in a particular embodiment, a compound capable of phosphorylating an MAPK protein (e.g., a kinase, etc.).

The phosphorylation of a protein as well as the determination of the effect of mimicking the phosphorylation on the MAPK protein can be determined by any conventional method known by the skilled person in the art. Various assays are known for determining the phosphorylation state of a protein, or the amino acid residue which is phosphorylated in a certain protein, such as for example *in vitro* kinase activity assays using radioactively labeled ATP; two-dimensional electrophoresis of the proteins thus phosphorylated and labeled (which allows analyzing how many amino acid residues are phosphorylated in a protein); mass spectrometry of the previously purified protein the phosphorylation state of which is to be measured; directed mutagenesis followed by *in vitro* kinase activity assay with the purified proteins; phospho-peptide analysis involving the separation in two dimensions of a phosphorylated protein after digestion by trypsin, or the least technically complicated, Western blot, which contemplates the use of antibodies against said protein which specifically recognize the amino acid residue or the epitope of the protein that is phosphorylated. The techniques for detecting phosphorylated residues in proteins are widely known by the skilled person in the art and are included in the state of the art.

In a particular embodiment, said MAPK protein is a p38 kinase, such as a mammal p38, and phosphorylation is carried out in the Thr123 residue present in said mammal p38, α isoform.

In another aspect, the invention relates to an antibody that is capable of binding to an MAPK protein of the invention, wherein said MAPK protein is a p38 protein, and/or able to detect said MAPK protein of the invention, wherein said antibody is able to specifically recognize the phosphorylated Thr123 amino acid residue or an epitope of said MAPK protein comprising the phosphorylated Thr123.

As used in this specification, the term "antibody" intends to include both chimeric or recombinant antibodies and monoclonal antibodies and polyclonal antibodies or proteolytic fragments thereof, such as fragments, Fab or F(ab')2, etc. Furthermore, the DNA encoding the variable region of the antibody can be inserted in other antibodies so as to produce in this way chimeric antibodies. Simple chain antibodies (scFv) can be polypeptides formed by simple chains having the characteristic ability of an antibody that binds to an antigen and comprising a pair of sequences of amino acids homologous or analogous to the light and heavy chain variable regions of an immunoglobulin (VH-VL or scFv bond). The polypeptides analogous to the light and heavy chain variable regions of an antibody can bind, if so desired, through a linker polypeptide. Methods for producing antibodies are well known by persons skilled in the art and are included in the state of the art.

By way of illustration, the antibody proposed by the invention is an antibody able of binding to and/or detecting an epitope present in said MAPK protein of the invention, wherein said MAPK protein is a p38 protein, and wherein said antibody is able to specifically recognize the phosphorylated Thr123 amino acid residue or an epitope of said MAPK protein comprising the phosphorylated Thr123. In a particular embodiment, said MAPK protein of the invention is a phosphorylated mammal p38 protein in a phosphorylation site that is different from the phosphorylation site or sites present in the activation segment of said mammal p38, for example, a phosphorylated mammal p38 in Thr123, or a fragment of said protein comprising said phosphorylated residue. In a specific embodiment, said antibody is able of binding to an epitope comprised in a fragment of the mammal p38 kinase, said fragment comprising a phosphorylated Thr123 residue, or a positionally equivalent (matching) residue in other MAPK p38 proteins. In another specific embodiment, said antibody is an antibody able of binding to the epitope comprising the amino acid sequence shown in SEQ ID NO: 2, an epitope that is highly conserved throughout evolution, therefore said SEQ ID NO: 2 can be considered to be a consensus sequence of said epitope among the homologous p38 proteins of the different species.

It is also disclosed a compound that is capable of binding to an MAPK protein of the invention which binds to said MAPK protein in the new phosphorylation site identified by this invention [i.e., Thr123 of mouse p38, α isoform, or a residue of a positionally equivalent amino acid in another MAPK protein as it is defined by multiple alignment of amino acid sequences], or at the area surrounding said site, said compound causing a decreased phosphorylation of the MAPK protein at the activation segment and thereby prevents its activation and/or its activity towards its substrates, for example, a compound which introduces a negative charge or a bulky residue either in said phosphorylation site or at the area surrounding said site. Illustrative, non limitative examples of said compound includes:
(i) a compound capable of binding to the docking region of p38 and able to mimic the introduction of a negative charge (e.g., a phosphate group) in said area, e.g., the introduction of a negative charge or a bulky residue at Thr123 (or at the surrounding area) of mouse p38, α isoform, or a residue of a positionally equivalent amino acid in another MAPK protein as it is defined by multiple alignment of amino acid sequences, and the association of said compound at said phosphorylation site Thr123, or at the area surrounding Thr123 prevents the activation of said MAPK protein; or
(ii) a compound capable of binding to the docking region of p38 and able to mimic the introduction of a negative charge (e.g., a phosphate group) in said area, e.g., the introduction of a negative charge or a bulky residue at Thr123 of mouse p38, α isoform, or a residue of a positionally equivalent amino acid in another MAPK protein as it is defined by multiple alignment of amino acid sequences, and the association of said compound at said phosphorylation site Thr123 impairs the activity of said MAPK protein towards its substrates.

It is also disclosed the use of said compound able of binding to an MAPK protein of the invention and/or able to detect said MAPK protein of the invention for analyzing the risk or predisposition of a subject of developing a pathology mediated by an active MAPK, or for evaluating or monitoring the effect of a therapy administered to a subject who has said pathology, or for analyzing the stage or severity and/or the evolution of said pathology, as well as in the identification of potentially useful compounds for the treatment of said pathology.

It is also disclosed a vector comprising:
(i) a nucleic acid sequence encoding a compound phosphorylating a phosphorylation site that is different from the phosphorylation site or sites present in the activation segment of an MAPK protein, wherein said different phosphorylation site is Thr123 of the mouse p38, α isoform, or a residue of a positionally equivalent amino acid susceptible of phosphorylation in another MAPK protein as it is defined by multiple alignment of amino acid sequences, and the phosphorylation in said different phosphorylation site prevents the activation of said MAPK protein; or
(ii) a nucleic acid sequence encoding a compound preventing the phosphorylation of a phosphorylation site that is different from the phosphorylation site or sites present in the activation segment of an MAPK protein; or
(iii) a compound phosphorylating a phosphorylation site that is different from the phosphorylation site or sites present in the activation segment of an MAPK protein, wherein said different phosphorylation site is Thr123 of the mouse p38, α isoform, or a residue of a positionally equivalent amino acid susceptible of phosphorylation in another MAPK protein as it is defined by multiple alignment of amino acid sequences, and the phosphorylation in said different phosphorylation site prevents the activation of said MAPK protein; or
(iv) a compound preventing phosphorylation in a phosphorylation site that is different from the phosphorylation site or sites present in the activation segment of an MAPK protein.

In a particular embodiment, said MAPK protein is a mammal p38 kinase and the phosphorylation takes place in a phosphorylation site that is different from the phosphorylation site or sites present in the activation segment of said mammal p38, for example, in Thr123 of a mammal p38.

The vector can be a viral vector or a non-viral vector, which are well known by persons skilled in the art and can be used in therapy, for example, in gene therapy.

In a particular embodiment, the vector comprises a nucleic acid sequence encoding a compound phosphorylating a phosphorylation site that is different from the phosphorylation site or sites present in the activation segment of an MAPK protein, wherein said different phosphorylation site is Thr123 of mouse p38, α isoform, or a residue of a positionally equivalent amino acid susceptible of phosphorylation in another MAPK protein as it is defined by multiple alignment of amino acid sequences, and the phosphorylation in said different phosphorylation site prevents the activation of said MAPK protein, or a compound phosphorylating said phosphorylation site that is different from the phosphorylation site or sites present in the activation segment of an MAPK protein, wherein said different phosphorylation site is Thr123 of mouse p38, α isoform, or a residue of a positionally equivalent amino acid susceptible of phosphorylation in another MAPK protein as it is defined by multiple alignment of amino acid sequences, and the phosphorylation in said different phosphorylation site prevents the activation of said MAPK protein. The phosphorylation in said different phosphorylation site produces the inhibition of the activity of said MAPK protein, therefore said vector of the invention can be useful for the treatment of pathologies mediated by active MAPKs.

In another particular embodiment, the vector comprises a nucleic acid sequence encoding a compound preventing the phosphorylation of a phosphorylation site that is different from the phosphorylation site or sites present in the activation segment of an MAPK protein or a compound preventing the phosphorylation of said phosphorylation site that is different from the phosphorylation site or sites present in the activation segment of an MAPK protein, such as for example a kinase inhibitor such as the GRK2 kinase inhibitor or a phosphatase dephosphorylating said phosphorylation site. Since the phosphorylation of said phosphorylation site is prevented, the activation of the MAPK protein can be promoted, which can be particularly interesting for treating cancer when the existence of active MAPK proteins after subjecting the subject to radiotherapy or chemotherapy leads to the death of tumor cells. Therefore, in this case, the vector of the invention can be useful for the treatment of pathologies mediated by active MAPKs, particularly cancer.

In a specific embodiment of the vector, the compound phosphorylating a phosphorylation site that is different from the phosphorylation site or sites present in the activation segment of an MAPK protein is a kinase, or a functionally active fragment thereof able to carry out the characteristic function of said kinase, for example, the GRK2 kinase or a functionally active fragment thereof, which, as is shown by this invention, phosphorylates the Thr123 residue present in mammal p38, such as mouse p38, isoform α.

In another aspect, the invention relates to a pharmaceutical composition comprising a therapeutically effective amount of an antibody able to bind to an MAPK protein and/or able to detect said MAPK protein, wherein said MAPK protein is a p38 protein selected from:
a) an MAPK protein comprising a phosphorylated residue in a phosphorylation site that is different from the phosphorylation site or sites present in the activation segment of said MAPK protein, or a fragment of said protein comprising said phosphorylated residue, wherein
   - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
   - the phosphorylation at said different phosphorylation site prevents the activation of said MAPK protein and also its activity towards its substrates; and
b) an MAPK protein comprising a negative charge or a bulky residue in a phosphorylation site, or at the area surrounding said phosphorylation site, that is different from the phosphorylation site or sites present in the activation segment of said MAPK protein, or a fragment of said protein comprising said phosphorylated residue, wherein
   - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
   - the introduction of a negative charge or a bulky residue at said phosphorylation site, or at the area surrounding said phosphorylation site, prevents the activation of said MAPK protein and also its activity towards its substrates, and
wherein said antibody is able to specifically recognize the phosphorylated Thr123_amino acid residue or an epitope of said MAPK protein comprising the phosphorylated Thr123, together with, optionally, a pharmaceutically acceptable carrier.

In a particular embodiment, said antibody is able to bind to an epitope comprised in a fragment of the mammal p38 kinase, said fragment comprising a phosphorylated Thr123 residue, or a positionally equivalent residue in other p38 MAPK proteins. In another embodiment, said antibody is able to bind to an epitope comprising the amino acid sequence shown in SEQ ID NO: 2.

For their administration in the prevention and/or treatment of a pathology mediated by an active MAPK, the active compounds (including the vectors) are formulated in a suitable pharmaceutical composition, in a therapeutically effective amount, together with one or more pharmaceutically acceptable carriers, adjuvants or excipients.

Examples of pharmaceutical compositions including any solid (e.g. tablets, capsules, granules, etc.) or liquid (e.g. solutions, suspensions, emulsions, etc.) composition for their administration by any suitable administration method, for example, oral, subcutaneous, intraperitoneal, intravenous, etc., typically administered orally due to the generally chronic character of the disease to be treated..

In a particular embodiment, said pharmaceutical compositions can be in an orally administered solid or liquid pharmaceutical form. Illustrative examples of orally administered pharmaceutical forms include, tablets, capsules, granules, solutions, suspensions etc., and can contain conventional excipients, such as binders, diluents, disintegrating agents, lubricating and wetting agents etc., and can be prepared by conventional methods. The pharmaceutical compositions can also be adapted for their parenteral administration in the form of , for example, sterile, lyophilized solutions, suspensions or products in the suitable dosage form; in this case, said pharmaceutical compositions will include the suitable excipients such as buffers, surfactants, etc. In any case, the excipients will be chosen according to the selected pharmaceutical administration form. A review of the different pharmaceutical administration forms and their preparation can be found in the book "Tratado of Farmacia Galénica", of C. Fauli i Trillo, 10th Edition, 1993, Luzán 5, S.A. de Ediciones.

Generally, the therapeutically effective amount (or vector) to be administered will depend, among other factors, on the subject to be treated, on the severity of the pathology suffered by said subject, on the chosen administration form, etc. For this reason, the doses mentioned in this invention must only be considered as guidelines for the person skilled in the art, and the doses must be adjusted according to the aforementioned variables. Nevertheless, the pharmaceutical composition of the invention can be administered one or more times a day, for example, 1,2 3 or 4 times a day, in a typical total daily amount comprised between 25 and 75 mg/kg/day.

The pharmaceutical composition of the invention can be used together with other additional drugs useful in the prevention and/or treatment of said pathologies mediated by active MAPKs for providing a combination therapy. Said additional drugs can form part of the same pharmaceutical composition or alternately, they can be provided in the form of a separate composition for its simultaneous or non-simultaneous administration with the pharmaceutical composition provided by this invention.

In another aspect, the invention relates to the use of:
(i) an antibody able to bind to an MAPK protein and/or able to detect said MAPK protein, wherein said MAPK protein is a p38 protein selected from:
   a) an MAPK protein comprising a phosphorylated residue in a phosphorylation site that is different from the phosphorylation site or sites present in the activation segment of said MAPK protein, or a fragment of said protein comprising said phosphorylated residue, wherein
      - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
      - the phosphorylation at said different phosphorylation site prevents the activation of said MAPK protein and also its activity towards its substrates; and
   b) an MAPK protein comprising a negative charge or a bulky residue in a phosphorylation site, or at the area surrounding said phosphorylation site, that is different from the phosphorylation site or sites present in the activation segment of said MAPK protein, or a fragment of said protein comprising said phosphorylated residue, wherein
      - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
      - the introduction of a negative charge or a bulky residue at said phosphorylation site, or at the area surrounding said phosphorylation site, prevents the activation of said MAPK protein and also its activity towards its substrates, and
   wherein said antibody is able to specifically recognize the phosphorylated Thr123_amino acid residue or an epitope of said MAPK protein comprising the phosphorylated Thr123; or
(ii) a GRK2 protein; or
(iii) a vector comprising a nucleic acid encoding GRK2;
in the manufacture of a pharmaceutical composition for the treatment of a pathology mediated by active MAPKs, wherein said MAPK protein is a mammal p38 protein and the pathology is aortic constriction, heart failure following advanced coronary disease, congestive heart failure or rheumatoid arthritis.

In a particular embodiment, said MAPK protein is a mammal p38 kinase and the phosphorylation takes place in a phosphorylation site that is different from the phosphorylation site or sites present in the activation segment of said mammal p38, for example, in Thr123 of a mammal p38.

In a particular embodiment, said antibody is able to bind to an epitope comprised in a fragment of the mammal p38 kinase, said fragment comprising a phosphorylated Thr123 residue, or a positionally equivalent residue in other p38 MAPK proteins. In another embodiment, said antibody is able to bind to an epitope comprising the amino acid sequence shown in SEQ ID NO: 2.

In a particular embodiment, said compound is the GRK2 kinase which phosphorylates Thr123 of mammal (mouse) p38, α isoform.

In another aspect, the invention relates to a kit comprising an MAPK protein of the invention, or an antibody able to bind to an MAPK protein and/or able to detect said MAPK protein, wherein said MAPK protein is a p38 protein selected from:
a) an MAPK protein comprising a phosphorylated residue in a phosphorylation site that is different from the phosphorylation site or sites present in the activation segment of said MAPK protein, or a fragment of said protein comprising said phosphorylated residue, wherein
   - said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
   - the phosphorylation at said different phosphorylation site prevents the activation of said MAPK protein and also its activity towards its substrates; and
b) an MAPK protein comprising a negative charge or a bulky residue in a phosphorylation site, or at the area surrounding said phosphorylation site, that is different from the phosphorylation site or sites present in the activation segment of said MAPK protein, or a fragment of said protein comprising said phosphorylated residue, wherein
   - said different phosphorylation site is the threonine residue in position 123 (Thr23) of mouse p38, α isoform, or a residue of a positionally equivalent amino acid susceptible of phosphorylation in another MAPK protein as it is defined by multiple alignment of amino acid sequences, and
   - the introduction of a negative charge or a bulky residue at said phosphorylation site, or at the area surrounding said phosphorylation site, prevents the activation of said MAPK protein and also its activity towards its substrates, and
wherein said antibody is able to specifically recognize the phosphorylated Thr123_amino acid residue or an epitope of said MAPK protein comprising the phosphorylated Thr123.

In a particular embodiment, said antibody is able to bind to an epitope comprised in a fragment of the mammal p38 kinase, said fragment comprising a phosphorylated Thr123 residue, or a positionally equivalent residue in other p38 MAPK proteins. In another embodiment, said antibody is able to bind to an epitope comprising the amino acid sequence shown in SEQ ID NO: 2.

The kit herein disclosed can be used in the diagnosis of a pathology mediated by an active MAPK, or for determining the risk or predisposition of a subject of developing said pathology, or for evaluating or monitoring the effect of a therapy administered to a subject who has said pathology, or for analyzing the stage or severity and/or the evolution of said pathology, as well as in the identification of potentially useful compounds for the treatment of said pathology. In a specific embodiment, said MAPK protein is a phosphorylated mammal p38 kinase in Thr123 of a mammal p38, isoform α.

It is also disclosed a method for the treatment of a pathology mediated by an active MAPK, wherein said MAPK protein is p38 protein, comprising the administration of a pharmaceutical composition provided by this invention to a subject in need of treatment.

The following example illustrates the invention and does not intend to limit the scope thereof.

### EXAMPLE

### Phosphorylation of the Thr123 of p38 protein by the GRK2 enzyme

### I. MATERIALS AND METHODS

### Products

All the reagents and products used are analytical grade. Sodium, calcium, ammonium, manganese and magnesium chlorides, sodium and potassium phosphates, sodium carbonates, sodium hydroxide, sodium acetate, sucrose, urea, Tris, formaldehyde, paraformaldehyde, glycine, glacial acetic acid, hydrochloric acid, ethanol, ethanol, butanol and glycerol were supplied by Merck. ATP (adenosine triphosphate), sodium fluoride, deoxycholic acid, EDTA (ethylenediaminetetraacetic acid), EGTA (ethylene glycol bis (2-aminoethylene ether)-N-N-N'-N'-tetraacetic acid), β-mercaptoethanol, DTT (dithiothreitol), heparin, sodium orthovanadate, DMSO (dimethylsulphoxide), Ponceau red, HEPES (N-(2-hydroxyethyl)piperazine-N'-2-ethanesulphonic acid), Nonidet P-40, Triton x-100, Tween-20, aprotinin, trypsin inhibitor, sodium azide, Protein A-Sepharose, were supplied by Sigma. PMSF (phenyl-methyl-sulphonyl fluoride), benzamidine, reduced glutathione, BSA (bovine serum albumin) and ampicillin and kanamycin antibiotics as well as IPTG (isopropyl-beta-D-thiogalactopyranoside) were obtained from Roche. TEMED (N,N,N,N-tetramethylethylenediamine), SDS (sodium dodecyl sulphate), ammonium persulphate, bromophenol blue, Coomassie blue, prestained protein standards with a known molecular weight, nitrocellulose paper and Bradford reagent were provided by Bio-Rad. Folin-Ciocalteau reagent was obtained from Panreac, TCA (trichloroacetic acid) from Carlo-Erba. The radioactive [γ-³²P] ATP isotope was provided by Amersham Biosciences and the methionine-cysteine metabolic marking mixture [³⁵S] was supplied by New England Nuclear.

### Constructs and plasmids used

The following plasmids have been used in this specification:
*GRKs*
   - The rat pCMV-GRK3 construct was given by Dr. S. Cotecchia, from Lausanne University, Switzerland.
   - The bovine pCDNA3-GRK2, bovine pCDNA3-GRK2-K220R and pCDNA3-GRK5 constructs were given by the laboratory of Dr. J. L. Benovic from Thomas Jefferson University in Philadelphia, U.S.A.
   - The pCEFL-DNAc antisense construct of GRK2 was sent by Dr. C. Shayo.
*p38 MAPK Module*
   - The constitutively active mutant pCDNA3-MKK6β(E) (pCDNA3-MKK6β (Glu): MKK6S207E/T211E has been provided by Dr. J. M. Redondo, from the Centro of Biologia Molecular Severo Ochoa *(Severo Ochoa Molecular Biology Centre)* (Madrid), who likewise provided the mouse pCDNA3-Flag-p38α construct and the pGEX2T- p38α prokaryotic expression vector.
   - The pGEX4T-Mxi2 and pGEX4T-Mxi2Δ17 vectors, intended for the production of fusion proteins with GST, were donated by Dr. P. Crespo and Dr. V. Sanz, of the University of Cantabria.
*Others*
   - The empty pCDNA3 vector is from Invitrogen.
   - The plasmid pCEFL-EGFP was provided by Dr. C. Murga (Centro of Biologia Molecular Severo Ochoa).
   - The pBC12BI-β₂-AR construct was given by Dr. A. Ruiz-Gómez (Centro of Biologia Molecular Severo Ochoa).
   - The Raf-1YY340/341DD mutant was provided by Doctor A. S. Dhillon, Beatson Institute for Cancer Research, Glasgow, U.K.
   - pTrcHisB was obtained from Invitrogen

### Cell Cultures

### Established cell lines

Several established cell lines have been used: HEK293 cells (human embryonic kidney) were obtained from Invitrogen, COS-7 cells (green monkey kidney cells) and Sf9 *(Spodoptera frugiperda)* cells were obtained from ATCC (American Type Culture Collection). The HEK293 and COS-7 cells were grown in monolayers on individual P-100, P-60 (Falcon) plates or multiwell M6, M12 or M24 (Falcon, Costar) plates in Dulbecco's Modified Eagle Medium (DMEM) supplemented with 2 mM glutamine, 10% fetal calf serum and a mixture of antibiotics (50 µg/ml gentamicin, 0.01% streptomycin and 0.063% penicillin G).

Likewise, two mass-cultures stably expressing GRK2, generated from EBNA (derived from HEK and transfected with a plasmid encoding the EBNA antigen) cells and from the neomycin-resistant pCDNA3-GRK2 vector, were used, therefore they were cultured in the presence of 200 µg/ml geneticin (neomycin G418, Calbiochem).

The preadipocytic cell line 3T3-L1, obtained from the ATCC, as well as the stable lines generated from it, were maintained in DMEM medium, supplemented with glutamine and antibiotics with 10% new born calf serum (NCS). 750 µg/ml geneticin was added to the populations stably transfected with pCDNA3-GRK2 and with pCDNA3-K220R. The conditions of differentiation into adipocytes are detailed below.

All these cell types were incubated at 37°C in a moistened atmosphere with 5-7% of CO₂.

The Sf9 cells were grown in suspension at a density of 3x10⁵ cells/ml with stirring at 150 rpm, or in monolayer on P-100 or P-150 plates in Grace's medium (Gibco) supplemented with fetal calf serum and gentamicin (50 µg/ml) at 27°C without a CO₂ atmosphere.

Primary cultures of murine pacrophages were obtained and maintained using standard protocols. Essentially, 3 month-old C57BL/6 GRK2 +/+ and +/- mice, kindly donated by Dr. Marc Caron (Duke University, North Carolina) were intraperitoneally injected with sodium thioglicolate (lml). Four days later, peritoneal macrophages were isolated by a 15 ml intraperitoneal wash with PBS. One million cells were seeded per well on an M 12 plate, allowed to adhere in RPMI medium supplemented with 0.5% FCS and washed extensively. The resulting macrophages were estimulated for 16 hours at 37°C in a humidified chamber with the detailed concentrations of LPS from E. Coli (Sigma) in RPMI 0.5% FCS.

### Transfections

The transient transfections of HEK293 and COS-7 cells were performed in P-100 P-60 plates at a confluence between 70 and 80% by the Lipofectamine/PLUS method, (Invitrogen). Although alternative transfection protocols were used with reagents such as Fugene (Roche), JetPei (Poly Transfection) or Escort-II (Sigma), the most used process was the lipofectamine process. In summary, a day before the transfection, 1.5 × 10⁶ cells (HEK293) were plated by P-60 or a number of cells correlatively proportional to the surface of the plate used. From this point onwards, the protocols refer to a P-60 plate. The following day, a mixture (1) of highly pure plasmidic DNA (isolated in affinity columns supplied by Quiagen and resuspended in sterile MilliQ water) (3-5 µg in the case of P-60), PLUS reagent (8 µl for P-60) and OPTIMEM (Gibco, BRL), (250. µl for P-60) was prepared which was incubated for 15 minutes at room temperature. In each experiment, the necessary amount of empty vector (generally, pCDNA3) was added so as to keep the total amount of DNA per plate constant. In a parallel way and in another tube, lipofectamine (12 µl for P-60) is mixed (2) with OPTIMEM (250 µl for P-60) and is also incubated. After 15 minutes, (1) and (2) are mixed in equal proportions, and the prepared mixture is incubated for another 15 minutes and it is finally poured on the plates (final reaction mixture: 0.5 ml for P-60), which has previously been covered with OPTIMEM (2 ml per P-60). The cells are incubated at 37°C for 3 hours in the transfection medium, after which the transfection medium is removed and substituted by DMEM medium supplemented with 10% serum. The following day, the medium is replaced with fresh medium and the cells are left to recover, at least for 24 hours before processing the culture for the experiment. Generally, the treatments, collection and lysis of the cells occurred 48 hours after transfection.

### GRK2 and p38MAPK

In the GRK2 and Flag-p38α association experiments, a 1:1:1 ratio of GRK2 (or GRK2-K220R), Flag-p38α, and β₂-adrenergic receptor was used (generally 1 µg of each per p60).

In the overexpression assays of increasing doses of GRK2, HEK293 cells, normally seeded in 6 or 12 (M6 or M12) multiwell plates, were transfected with pCDNA3-Flag-p38a, pCDNA3-MKK6_{CAM}, and with increasing amounts of the pCDNA3-GRK2 vector (shown in the Figures). Generally, 100 ng of Flag-p38α, 100 ng of MKK6_{CAM}, and 0 to 1 µg of GRK2 were used for an M6. In all the points, the total amount of DNA was completed with pCEFL-EGFP and with empty pCDNA3, in substitution of pCDNA3-MKK6_{CAM}, in the case of control points.

In the GRK2 antisense DNA transfections in M6 multiwell plates, the amounts of DNA used were somewhat different: 150 ng of pCDNA3-Flag-p38α, 50 ng of pCDNA3-MKK6_{CAM}, and, 0.5 µg to 2 µg of the pCEFL-GRK2 antisense (AS) vector or the same amounts of pCEFL-EGFP. In all the points, the total amount of DNA was completed with the empty pCEFL vector.

In analogous overexpression assays, the cells in M6 were transiently transfected, and always in duplicate, with: 150 ng of pCDNA3-Flag-p38α WT, or pCDNA3-Flag-p38α T123D and 50 ng of pCDNA3-MKK6_{CAM}, (or empty pCDNA3).

The transient expression of the different proteins was confirmed by analyzing the cell lysates (approximately 10% of the total volume of the cell lysate) by immunodetection after electrophoresis (Western blot), with specific antibodies as specified in each case.

### Cell treatments

The stimulation treatments of transiently transfected cells were carried out in all cases 48 hours after transfection. After stimulating with different agents, the cells were washed in cold phosphate buffer saline (PBS) and collected with the help of a scrapper. The lysis buffer in which they are collected depends on the specific immunoprecipitation that is to be carried out (see immunoprecipitation section).

The stimulation of HEK293 cells with 10 µM isoproterenol (Sigma) was carried out at 37°C in culture medium without serum. In these experiments, the cells were maintained without serum (serum-starved) for about 2 hours before stimulation for the purpose of minimizing the stimuli from compounds present in the serum.

The stimulation with 0.5 M NaCl (Merck) was carried out for 15 minutes in a cell incubator.

### Adipocyte Differentiation

The cell culture was carried out with 10% DMEM medium of NCS serum. However, the entire differentiation process which is described below must be performed in depleted AXC serum (ion exchange resin) by means of successive adsorptions of fetal calf serum on an anion exchange resin and on active carbon. AXC serum was provided by the kitchen service of the Instituto of Investigaciones Biomédicas *(Biomedical Research Institute).* The cells are grown until their confluence and are plated (5 x 10⁵ in P-100) in DMEM-10% AXC medium, supplemented with 4 µM of biotin (Sigma). The following day, the medium is replaced with fresh medium. The cells are grown another three days, until confluence is reached again, that day is called day "0". On day 0 of differentiation, the cells are cultured in a medium containing: 0.5 µM dexamethasone (Sigma), 0.5 mM 3-isobutyl-1-methylxanthine (IMBX, of Sigma) and 1 µM insulin (Sigma) and in which they will remain for another three days. On day 3, the medium of this adipogenesis initiating treatment is replaced by DMEM-10% AXC, supplemented with 4 µM biotin and 1 µM insulin, in which the remaining differentiation will take place, the medium being replaced every three days. From day 6 to day 15, the adipogenesis was analyzed by staining with Oil Red, a red coloring of lipophilic constitution which binds to the drops of fat accumulated by the adipocytes in their cytoplasm. To that end, the cells are fixed with formalin (3.7% formaldehyde) for 5 minutes and washed with cold PBS. They are incubated with a previously filtered 60:40 (v/v) Oil Red (dissolved in 0.2% isopropanol w/v) and water solution. They are abundantly washed with PBS and the cells are visualized under an optical microscope. The cells are counted in a total of 25 fields per each experimental plate.

### Mutant generation

### Point mutants

Most mutants were generated by means of the Stratagene QuickChange directed mutagenesis protocol. In summary, the anti-parallel mutagenic oligonucleotides- which are indicated below for each particular mutant- with which the polymerase chain reaction (PCR) was carried out in a thermal cycler (Applied Biosystems Gene Amp® 9700), were made by using thermostable Pfu as the polymerase. The integrally amplified vectors were digested with DpnI to remove mould or parenteral DNA and the digestion product was transformed into competing bacteria, from which the mutation incorporation could be checked by sequencing in the SIDI (Servicio Interdepartamental of Investigación, *Interdepartmental Research Service*) with specific priming oligonucleotides for each type of plasmid (Sp6, T7, T3, or the specific ones for sequencing cloned proteins in pGEX vectors).

### p38T123A

-oligonucleotide FWD: 5' GTG AAG TGC CAG AAG CTG GCC GAC GAC CAC GTT CAG 3' (SEQ ID NO: 3)
-oligonucleotide REV: 5' CTG AAC GTG GTC GTC GGC CAG CTT CTG GCA CTT CAC 3' (SEQ ID NO: 4)

The mutagenic PCR products were sequenced with the priming nucleotides SP6 and T7 lining the ORF (open reading frame) of p38 in pCDNA3 or with the specific nucleotides for sequencing the Amersham pGEX plasmid series.

### p38T123D

-oligonucleotide FWD: 5' GTG AAG TGC CAG AAG CTG **GAC** GAC GAC CAC GTT CAG 3' (SEQ ID NO: 5)
-oligonucleotide REV: 5' CTG AAC GTG GTC GTC **GTC** CAG CTT CTG GCA CTT CAC 3' (SEQ ID NO: 6)

### Truncated mutants

The truncated protein GST-280-360p38, corresponding to the last 80 amino acids of p38α was generated using the Invitrogen Gateway system. This polyvalent cloning method by recombinases allows the expression of the protein or the protein fragment of interest in a large number of plasmids for eukaryotic and prokaryotic hosts and with several epitopes.

Firstly, it was necessary to design the oligonucleotides allowing the incorporation of the attB sequences, target of the recombinases, lining the ORF region of p38α that is desired to be translated. They are called GTW, from Gateway. The oligonucleotide GTW-FWD was made such that the first amino acid of p38 to be translated (Ala 281, underlined in the nucleotide sequence) was in phase with the attB1 sequence. The oligonucleotide GTW-REV incorporates the termination codon of the translation (also underlined). The plasmid pGEX2T-p38α was used as a mould in PCR.
- oligonucleotide GTW-FWD: 5' GGG GAC AAG TTT GTA CAA AAA AGC AGG CTT CGC TGT CGA CCT ACT GGA GAA GAT G 3' (SEQ ID NO: 7)
- oligonucleotide GTW-REV: 5'GGG GAC CAC TTT GTA CAA GAA AGC TGG GTC TCA GGA CTC CAT TTC TTC TTG GTC 3' (SEQ ID NO: 8)

The cloning of the 240 bp PCR product into the pDONOR^{™}201 vector was carried out by means of the BP-clonase reaction (for at least one hour at 25°C) consisting of the recombination, mediated by the attB sequences, of the PCR product with the pDONOR vector. The reaction was stopped by adding Proteinase-K 10 minutes at 37°C. The DNA, product of the recombination, is used to transform DH5α bacterias, selected with kanamycin.

Subsequently, the intended plasmid was chosen: PEST15 assuring the prokaryotic expression of the protein cloned in it, as fusion, in phase from the recombination sequences to GST. (The C-terminal fragment of p38 was also introduced in the eukaryotic expression plasmid pDEST27, but said results have been omitted). The LR-clonase reaction was carried out by using the entry clone (pDONOR-280-360p38) and the vector: linearized pDEST15 , and incubating with the enzymatic LR-clonase mixture for 1 hour at 25°C. The reaction was stopped by incubating the sample with Proteinase-K 20 minutes at 37°C and, after the relevant checking by sequencing with attB oligonucleotides, the obtained DNA was used to transform BL21 bacteria, from which the GST-280-360 p38 construct was purified.

### DNA subclonings

For the expression and purification of MAPKAPK2 (MK2) with a C-terminal histidine tag, the plasmid pFtx5-MK2ΔN1, deleted in proline-rich N-terminus region for more stable expression, was obtained from Dr. Phil Cohen (University of Dundee, Scotland, UK) and used as a template in a PCR reaction using the primers:
5' GGG GCC **ATG G**TC AAG TCC GGC C 3' (SEQ ID NO: 9) and
5' CCCC **CTC GAG** GTG GGC CAG AGC CGC AGC 3' (SEQ ID NO: 10).

The product was subcloned NcoI-XhoI (in bold) in the pTrcHis2B vector (Invitrogen).

### Purified recombinant proteins and polypeptides.

Activated MEKK6/MEKK3 was provided by Upstate.

The purification of GRK2 was carried out by Dr. A. Ruiz-Gómez from Sf9 cells infected with GRK2 constructs in baculovirus.

Rhodopsin was purified from bovine retinas according to conventional methods. A preparation is thus obtained in which rhodopsin is more than 90% of the protein (evidenced by Coomassie Blue STAINING).

Fusion proteins GST-p38, GST-ATF2, GST-MEF2A, GST-Mxi2, GST-MxiΔ17 and GST-280-360p38, GST-p38T123A and p38T123D were purified according to conventional methods that are briefly described: said constructs are transformed into *E. coli* bacteria and their expression is induced with IPTG. The bacteria are sedimented and lysed in 10 mM Tris-HCl pH 8, 1% TritonX-100, 2 mg/ml lysozyme and protease inhibitors, after which the bacterial lysate is sonicated and clarified. It is loaded into the glutathion-Sepharose4B® (AmershamBiosciences) column and it is passed for a minimum of 3 hours, the column is washed with PBS and afterwards, the proteins are eluted with 50 mM Tris-HCl pH 8, 5 mM reduced glutathion (Sigma). The purity and the concentration of proteins obtained are checked in denaturing polyacrylamide-SDS gels. The origin of the plasmids encoding these proteins is specified in the previous section or, if they are generated by the inventors, they are assigned where appropriate.

The specific p38 substrates such as MBP (myelin basic protein) or PHAS-I (Phosphorylated, Heat and Acid Stable-regulated by Insulin) were obtained from SIGMA and from Stratagene, respectively. The APRTPGGRC peptide, described as a specific substrate of MAPKs used in phosphorylation reactions with the p38 kinase, was synthesized by the Servicio Proteómica (Proteomic Service) of CBMSO. It was dissolved to a final concentration of 0.5 mM in Tris 20 mM at pH 7.6.

Protein determination was carried out by the Bradford method or by the method of Lowry et al. using bovine serum albumin as a standard for constructing the standard line.

### Electrophoresis

### SDS-polyacrylamide gel electrophoresis

### Unidimensional gels

SDS-polyacrylamide gels were used according to the method described by Laemmli the arcrylamide-bisacrylamide percentages of which ranged between 7 and 12% according to the resolution required by the experiment. The following proteins were used as molecular weight standards: myosin (200 kDa), β-galactosidase (116.25 kDa), phosphorylase B (97.4 kDa), bovine serum albumin (66.2 kDa), ovoalbumin (45 kDa), carbonic anhydrase (31 kDa), soy trypsin inhibitor (21 kDa) and lysozyme (14 kDa) (Rainbow Markers, of Bio-Rad). In several cases, the gels were stained with Coomassie blue. After the proteins were resolved, the gel can be subjected to autoradiography if the proteins are marked with [γ-³²P]ATP, or a fluorography if the proteins are marked with [³⁵S]-methionine. In both cases, a fixing step is required in methanol:acetic acid (50:10) for 20 minutes. In metabolic markings, the signal was frequently amplified by incubating for 20 minutes with Amplify (Amersham), and then the gel was dried and exposed to an Agfa Curix RP2 X-ray film of 100 NIF.

### Two-dimensional gels

With the purpose of analyzing the number of substrate residues of phosphorylation by GRK2 in p38, both proteins were incubated in reaction conditions that are specified below. The resulting phosphoproteins were resolved in two-dimensional gels. The isoelectric focusing or first electrophoretic dimension was made by using a resolutive mixture of ampholytes (Bio-Rad), with a final pH range of 3-10, in a 4% acrylamide-bisacrylamide gel with 8 M urea. Occasionally, the first dimension was alternately carried out using the pre-assembled strips of Biorad (IPG Strips, with a pH range of 3-10). The second dimension was carried out in an 8% gel SDS-PAGE, and the phosphoproteins were detected by autoradiography.

### TAE-agarose gel electrophoresis of nucleic acids

The separation of DNA fragments was carried out in 0.8-1% horizontal agarose gels. The electrophoresis buffer used was TAE (40 mM Tris-acetic acid, 2 mM EDTA) and the charging buffer of the samples was 50% glycerol, 0.4% bromophenol blue and 0.4% xylene blue. The molecular weight standards were the fragments of enzymatic digestion with HindIII of the phagocytes and Φ29 (supplied by the fermentation service of the Centro de Biologia Molecular (Molecular Biology Centre)).

### Proteomic Sequencing

The determination of the location of the post-translational modification of interest was carried out by the Servicio de Proteómica del Nodo UAM (Universidad Autónoma of Madrid) (UAM Node Proteomic Service) of the Cardiovascular Network, included within the Servicio de Proteómica del Centro de Biología Molecular "Severo Ochoa" (http://www.cbm.uam.es/mkfactory.esdomain/webs/CBMSO/plt_Servicio_Pagina.aspx?Id Servicio=29&IdObjeto=118).

The samples are electrophoretically separated (SDS-PAGE 8%) and the gel is stained. The bands to be analyzed are excised form the polyacrylamide gel and subjected to tryptic digestion (trypsin of Promega).

The mixture of tryptic peptides was analyzed by MALDI-TOF: (Matrix-Assisted Laser Desorption/lonization- Time Of Flight, Autoflex model of Broker). As a summary, the peptide species are adsorbed by crystallization to a matrix, after which they are unbound in a protonated from by the incidence of short pulses from a laser. This method of sample ionization is coupled to an analyzer of time of flight. Effectively, the mono-loaded peptides acquire a kinetic energy proportional to their mass, and "fly" through a vacuum tube until they impact the detector. A small aliquot (0.5 µl) of the supernatant of the digestion was directly analyzed in a mass spectrometer of the MALDI-TOF type, autoflex model of Bruker, equipped with a reflector, using DHB (2,5-dihydroxybenzoic acid) as a matrix and an Anchor-Chip surface (Bruker) as a sample holder. The spectrum obtained finally corresponds to the peptides separated according to the mass-charge ratio (m/z). The fragmentation spectra of GST-p38 and of GRK2-phosphorylated GST-p38 were compared and a candidate peptide was found.

To verify this indication, the samples were subjected to another type of spectrometric analysis which allows obtaining fragmentation spectra (MS/MS) of individual peptides: ElectroSpray/Mass Spectrometry- Ionic Tramp ES/MS-IT, Deca-XP model of Thermo-Finnigan, San José, California, USA). Before the ionization and because the latter can be carried out in a capillary, i.e. with liquid samples, the candidate peptide (previously "suspected" by MALDI-TOF) was separated by means of RP-HPLC (reversed-phase high pressure liquid chromatography). A column with a internal diameter of 180 µm (0.18 mm x 150 mm BioBasic 18 RP column of Thermo-Keystone) was used at a flow of 1.5 µl/m in micro-spray mode with a "metal needle-kit" interface (Thermo-Finnigan), with a gradient of 5% to 60% of solvent B for the elution (90 minutes) of the peptides. The chromatography is coupled to the ionic tramp mass spectrometer. In this fragmentation process, the samples are subjected to an intense electric field, as a result of which charged drops are generated which, after solvent evaporation, end up emitting ions corresponding to the peptides of the sample mixture. These can be multiprotonated, preferable in the N-terminal end and in the residues of histidine, arginine and lysine. The ionic tramp analyzer generates a three-dimensional electric field which allows separating the ions from ionization by electrospray. Thus, a fragmentation spectrum or MS/MS spectrum is finally obtained which, when working in "SIM" mode (single ion monitoring), is limited to the fragmentation spectrum of the candidate peptide. The samples are analyzed in high sensitivity mode or "SIM" mode, monitoring the following m/z: 937.51 and 977.51. After theoretically predicting the fragmentation series of the "supposedly" phosphorylated peptide, several ferments of the series b and y" are assigned to the obtained spectrum.

### Immuno-protocols

Table I shows the primary antibodies used.

### Immunodetection after electrophoresis ("Immunoblot or Western blot")

The samples for analysis (purified proteins, lysates or sub-cell fractions, etc.) are resolved in SDS-polyacrylamide gels together with commercial molecular weight standards (Bio-Rad). The proteins thus separated are transferred to a nitrocellulose filter (Bio-Rad Transblot) by liquid transference in carbonate buffer (3 mM Na₂CO₃,10 mM NaHCO₃, 20% methanol pH approximately 10) for 75 minutes (at 50 V in the case of 12 x 14 cm gels using a Bio-Rad Trans-Blot Cell or at 30 V for 120 minutes). After staining the nitrocellulose membrane with Ponceau red, it was incubated overnight at 4°C in TBS medium (10 mM Tris-HCl pH 7.5, 150 mM NaCl) supplemented with 5% skimmed milk powder (Molico) at 5% or BSA at 5% , with the aim of blocking the possible unspecific binding sites. After rejecting the blocking medium, the membrane is put into contact with the corresponding antibody, diluted (Table I) in 1% TBS-BSA. Before incubating with the second antibody (rabbit anti-immunoglobulin bound to peroxidase, when the first body is polyclonal and mouse anti-immunoglobulin for monoclonal bodies, both of Nordic Immunology) diluted 1:50,000, the membrane is washed three times (3 x 10 minutes) with con TBS-Tween 20 to 0.15%. Finally, for the developing, a chemoluminiscent method is used in which the peroxidase catalyzes the oxidation of the luminol substrate in the presence of H₂O₂ (ECL, Amersham). The quantification was carried out by laser densitometry of the exposed films (Molecular Dynamics 300A Computing Densitometer).

The polyclonal Anti-phospho-Thr123p38 serum was generated in rabbits by Pacific Immunology using the peptide QKL pT DDHVQFLIYC from murine p38α as immunogen and subsequently purified by two serial passages through peptide and anti-phospho peptide affinity columns. The anti-His antibody was purchased from Sigma.

### Determination of the activity of p38 and of ERK

The determination of the degree of activity of transfected p38 and of transfected ERK caused by different activating stimuli was carried out by an immunodetection method. Specific antibodies reacting only with the phosphorylated and active of these kinases form (anti-phospho p38 and anti-phosphoERKs respectively (see description in Table I).

The HEK293 cells, generally subjected to starvation (medium without serum) of a variable duration: 2 hours in the case of p38 and all night in the case of ERK, were stimulated and, after processing the lysate cells, the immunodetection of the phosphoproteins was carried out with the phospho-specific antibodies of the activation segments of both proteins. After developing this first immunodetection, the immune complexes were released with the buffer: 2% SDS, 100 mM β-mercaptoethanol, 62.5 Mm TrisHCl, pH 6.7 and the re-incubation of the membranes with the total anti-p38 or anti-ERK antibodies was carried out. The quantification of the bands was carried out in a laser densitometer. In all the cases, the values obtained for the bands detected with the anti-phospho-protein antibody were normalized in relation to the total amount of p38 or ERK expressed in the cells. In this way, the increase in stimulation of the different kinases with respect to the baseline conditions is represented. In some cases, however, given the risk of the first developing interfering in the second one, the activation was evaluated by means of the densitometry of two different membranes, developed separately, one with the phospho-specific antibody and the other with the antibody against total protein.

### Immunoprecipitation

The total samples or cell lysates that will be subjected to immunoprecipitation are diluted in different buffers supplemented with protease inhibitors (STI Soybean Trypsin Inhibitor) and benzamidine 100 µg/ml, PMSF 200 µg/ml and aprotinine 10 µ/ml). The buffers varied according to the antibody used in each case, as specified below. In all the cases, after allowing the lysis to take place for a minimum of I hour at 4°C, with stirring, the samples were centrifuged (24000 xg) and aliquots were taken (approximately 10%) to confirm the expression of specific proteins. BSA (500 µg per p60) and the corresponding amount of antibody for each case were added to the remaining sample. All the immunoprecipitations were carried out at 4°C all through the night. On the following day, 30 µl of 50% protein A-Sepharose (Sigma) or the protein G-Sepharose (Zymed) was added according to whether the antibody was polyclonal or monoclonal, respectively, and it was incubated at 4°C for 90 minutes more. This step was omitted when the antibodies were covalently bound to the resins, in which case 5-10 µl of "immuno-resin" was added. The immune complexes were collected by centrifugation and after rejecting the supernatant, they were washed 3-5 times (800 xg, 5 minutes) with 10-15 ml of washing buffer.

When the destination of the immunoprecipitates was phosphorylation reaction, these were washed two more times (2 x 10-15 ml) with the incubation buffer of the same, without ATP.

The immunoprecipitated proteins were resuspended in electrophoresis breaking buffer and generally, they were boiled for 5 minutes for later loading the complete sample in an SDS-polyacrylamide gel of a suitable percentage.

### RIPA (radioimmunoprecipitation assay) buffer

This solubilization buffer was widely used, especially in the immunoprecipitation of GRKs: 300 mM NaCl, 20 mM Tris-HCl pH 7.5, 2% Nonidet P-40, 1% deoxycolic acid and 0.2% SDS.

### "M2 anti-flag" Immunoprecipitation buffer

For immunoprecipitations with the M2 anti-flag antibody, the cells were collected in: 10 mM sodium phosphate buffer pH 7.4 (prepared from parent solutions of 0.1 M NaHPO₄ and NaH₂PO₄), 150 mM NaCl and 1% n-dodecyl-β-D-maldoside. After lysis, it was completed to 300 µl (per p60 plate) with the saline buffer formed by 20 mM Tris-HCl; 150 mM NaCl and protease inhibitors. Finally, the immunoprecipitates were washed in the buffer formed by: 50 mM Tris-HCl pH 7.5, 20 mM MgCl₂, 1% Triton X-100, 1 mM EDTA, 1 mM MgCl₂, 15 mM NaF, 20 mM Pyrophosphate.

### Pull-down experiments

The proteins GST, GST-p38wt, GST-p38T123A and GST-p38T123D were bacterially expressed and isolated using Gluthatione-Sepharose 4B (GE-Amersham) following standard procedures (Murga, C. et al. High affinity binding of beta-adrenergic receptor kinase to microsomal membranes. Modulation of the activity of bound kinase by heterotrimeric G protein activation. J Biol Chem 271, 985-994 (1996)). His-MAPKAPK2 was purified using Probond resin (Invitrogen) following manufacturer's indications. MKK6_{CAM} was purchased from Upstate Biotech. The amount of proteins detailed in the legend to each figure were incubated in binding buffer (25 mM Tris pH 7.5, 0.25 M NaCl, 10 mM MgCl₂, 5 mM NaF and 0.5% BSA) for 30 min at 30°C with constant shaking. ATP (50 µM) was added for MAPKAPK2 pull downs. Precipitates were washed three times (10ml) with the same buffer containing 0.5% Triton X100. Precipitated complexes were resolved by SDS-PAGE and developed by Western Blot.

### Phosphorylation assays

### Phosphorylation in vitro of recombinant proteins

### p38 Phosphorylation by GRK2

Recombinant GST-p38 and GRK2 (both of them equimolar at 25-150 nM, except in the experiments for calculating kinetic parameters in which the concentrations are specified) were incubated in the p38 phosphorylation buffer (25 mm Hepes pH 7.5, 10 mM magnesium acetate, 50 µM ATP, 2000-3000 cpm/pmol [γ-32P] ATP) in a final volume of 40 µl. Normally, the phosphorylation reactions are left to take place for 30 minutes at 30°C. In the case of two-dimensional electrophoresis or of the samples intended for proteomic sequencing, the reaction extends to 1 or 2 hours.

The compounds heparin and SB203580 (Calbiochem), GRK2 and p38 inhibitors respectively, were used at concentrations ten times the IC₅₀ in order to ensure the complete inhibition of the respective kinases, that is, at 1.5 µM for heparin and at 0.5 µM for the SB. The substrates used were MBP (14 µg per point) or PHAS-I at a final concentration of 25 ng/µl for p38 and caseine (7.5 µg per point) for GRK2.

Sample processing after stopping the phosphorylation is identical to the foregoing cases, except when the protein resolution is carried out in an 8% gel (SDS-PAGE)

Proteins of fusion to GST: p38α, Mxi2, Maxi2Δ17 and 280-360 p38 (0.5 µg of each of them) were incubated with recombinant GRK2 (200 nM), in phosphorylation buffer (25 mM Hepes pH 7.5, 10 mM magnesium acetate, 50 µM ATP, 2000-3000 cpm/pmol [γ-³²P] ATP) for 30 minutes at 30°C. Heparin (150 nM) was included as a specific GRK2 inhibitor. The reactions were stopped by adding a breaking buffer with SDS. The samples were resolved with 8% SDS-PAGE. With the purpose of assuring the inclusion of identical amounts of protein, they were first visualized in the gel by Coomassie Blue staining. Subsequently, the gel was dried and the radioactivity incorporated to the proteins (³²P) was detected.

The precise p38 mutants in the hypothetical site of phosphorylation by GRK2 (T123) were generated and purified as described previously and subjected to phosphorylation by GRK2 in a p38 phosphorylation buffer (25 mM Hepes pH 7.5, 10 mM magnesium acetate, 50 µM ATP, 2000-3000 cpm/pmol [γ-32P] ATP) in a final volume of 40 µl. The relative concentration (10-80 nM) of GRK2 and of the p38 isoforms was varied as shown in the drawings.

### p38 Phosphorylation by MKK6_{CAM}

Phosphorylation assays (25 mM Hepes pH 7.5, 10 mM magnesium acetate, 15 mm NaF, 50 µM ATP and 1000-2000 cpm/pmol [γ³²P]ATP) were carried out *in vitro* with the fusion proteins GST-p38 WT, GST-p38 T123A and GST-p38 T123D (150 nM) as phosphorylation substrates of recombinant MKK6_{CAM} (40 ng) (Upstate Biotechnology). As in the previous cases, the reactions were left to take place at 30°C for 30 minutes and the proteins were resolved in 8% SDS-PAGE gels. With the purpose of assuring the inclusion of identical amounts of protein, they were visualized in the gel by Coomassie Blue staining. Subsequently, the radioactivity incorporated in each p38 isoform was determined.

### APRTPGGRR peptide phosphorylation by p38

HEK293, Flag-p38alpha cells were immunoprecipitated with M2Anti-Flag-agarose. The immunoprecipitates were washed three times with 10-15 ml of M2 buffer and two times with the same volumes of phosphorylation balancing buffer (15 mM NaF, 25mM Hepes pH 7.5 and 10 mM magnesium acetate). In the last wash, the immune-agarose complexes were resuspended in 1 ml of buffer and 10% of each point was separated (100 µl) in order to control the immunoprecipitaion of Flag-p38. Kinase-assays were carried out with the remaining immunoprecipitated Flag-p38, using the APRTPGGRR peptide as a substrate. The reactions were carried out in a final volume of 25 µl, in a phosphorylation buffer formed by 25 mM Hepes pH 7.5; 10 mM magnesium acetate, 15 mM NaF, 50 µM ATP and 500-1000 cpm/pmol [γ-³²P]ATP and 1-2 mM of the substrate peptide. When it is specified, SB203580 is added to the *in vitro* at a final concentration final of 0.5 µM. The phosphorylation is allowed to take place for 30 minutes at 30°C, after which it is stopped by adding 15 µl of 30% TCA. The proteins are precipitated by centrifugation (25,000 xg, 15 minutes, 4°C) and the supernatant containing the phosphorylated peptide is collected from each reaction. Square (1 cm x 1 cm) Whatman P81 paper cut-outs were impregnated with the peptide in solution. They were left to dry, were abundantly washed with 75 mM phosphoric acid and the radioactivity incorporated by the adsorbed peptide was finally quantified by Cerenkov. The p38 activity on that peptide refers, in each case, to the baseline (or background) activity detected in the points corresponding only to the peptide.

### Phosphorylation of the substrates of ATF2 and MEF2A by p38

The ATF2 and MEF2A forms bound to GST were used to test the catalytic activity of GST-p38. In most cases, 2 µg of GST-ATF2 or GST-MEF2A, of own production, were used. The phosphorylation conditions are the same as set forth in the foregoing cases. Nevertheless, in other occasions, relevantly pointed out in the Figures, 0.2 µg of substrate were used and the phosphorylation reaction was allowed to take pace for 15 minutes only.

### Sequence comparisons

*Alignments of the p38 orthologues and isoforms.*

The multiple alignments occurring were made with the program ClustalX (http://www-igbmc.u-strasbg.fr/BioInfo/ClustalX/) and manually adjusted by means of introducing gaps by Dr. Perdiguero, del Centro Nacional of Investigaciones Oncológicas, (National Centre for Oncological Research), Madrid.

### Mathematical and Statistical analysis of the data

The experiments were carried out for a minimum of two times and generally, the points were carried out in duplicate or triplicate. The data were expressed as the mean with the standard deviation of the mean (±SEM).

The Michaelis-Menten behaviour of the kinases was assumed for calculating the kinetic constants of the enzymatic reactions. The graphs were made with the "Kaleidagraph" program, provided with an algorithm capable of deducing the kinetic parameters: Michaelis-Menten constant (Km) and maximum speed (Smax=So (nmol of PO₄³⁻ incorporated.mg of enzyme⁻¹minute⁻¹)

The statistical analysis was carried out by means of the "two-sided Student's t-test" (two-tailed) and n-1 degrees of freedom where the null hypothesis is that there is no significant difference between the situation or condition, the statistical significance of which we wish determine and the baseline or control situation. The value corresponding to the probability of complying with the null hypothesis (p) obtained in each case ranged from p<0.001 to p<0.0001, as shown in the Figures.

### II. RESULTS

### Functional interrelations between GRK2 and p38MAPK.

### p38 is phosphorylated by GRK2

The inventors have studied the mechanisms controlling the modulation of the activity of GRK2 and its expression by MAPK, given the predominant involvement of GRK2 and its substrate receptors both in cardiac physiology and in the ethiology of cardiovascular diseases such as hypertension, congestive heart failure or angina pectoris; and given the importance of the p38 MAPK module in the development of the myocardium and its subsequent function. There is an inverse correlation between the increased levels of GRK2 and the inactivation of p38 in congestive heart failure. Furthermore, the levels of GRK2 are decreased in inflammatory-type disease such as for example, rheumatoid arthritis.

Subsequently, the inventors decided to study the possible interactions between GRK2 and p38. The first experimental approach was the phosphorylation assays of both proteins. Figure 1, panel A, shows that GRK2 phosphorylates p38. In order to reject the possibility of its being an autophosphorylation of p38 caused in some way by the presence of GRK2, heparin (16 ng/µl), which is widely used as a GRK2 inhibitor, was included in the phosphorylation assays having ascertained beforehand that heparin des not affect the catalytic activity of p38. The same panel shows that the catalytic activity of GRK2 entails a smaller phosphorylation of p38 therefore the activity of the Ser/Thr kinase of GRK2 is directly responsible for the radioactive mark incorporated in GST-p38. These experiments were completed with negative controls of GST phosphorylation by GRK2 and by inspecting the binding area between the GST portion and the ORF of p38.

A recent publication describes the autophosphorylation of p38 stimulated by its interaction with TAB1; the pyridinylimidazole SB203580, a competitive inhibitor for the specific ATP of p38, was included in phosphorylation assays. Panel B of Figure 1 shows the effect of the SB203580 inhibitor in the catalytic activity of p38 on a generic substrate of the latter such as MBP (myelin basic protein). Given that the baseline activity of p38α is trivial, a great phosphorylation of MBP is not observed and even so, the inhibition by SB203580 is detected. The two following lanes report that pyridinylimidazole does not affect the kinase activity of GRK2. Finally, the two last lanes of the autoradiography show that the autophosphorylation activity of p38 is increased in the presence of GRK2. The final conclusion of these described experiments is that p38 is phosphorylated *in vitro* by GRK2.

In order to check that phosphorylation was not taking place in the residues that can be phosphorylated by the p38-activating kinases (MAPKK) such as MKK3 and MMK6, the phosphorylation reactions were carried out in cold conditions (in other words without, sin [γ-³²P]-ATP) and the immunodetection was carried out using the phosphospecific antibody of Anti-P-p38 (Figure 1, section C). This antibody recognizes phosphorylate epitopes in the sequence in the threonine 180 or in the tyrosine 182 of p38. As can be observed in the lower panel, only the presence of the recombinant protein MKK6 gives rise to the recognition of phospho-p38 by the mentioned antibody. In the upper panel, the total proteins were detected by an anti-GRK2 antibody recognizing the GST (to see GST-p38). These experiments show that GRK phosphorylates at p38 in a residue different from the T180.

### GRK2 phosphorylates p38 quickly and with high affinity

The study of the kinetic parameters of the reaction shows that these indicate that the reaction takes place *in vivo.* Figure 1 shows the kinetic characterization of the phosphorylation. Initially, experiments of time course reactions (panel D) are carried out in which it is shown that the phosphorylation is detected quickly (after 5 minutes), estimating an average time of 15 minutes for reaching 50% of the maximum phosphorylation. GRK2 is an eclectic kinase capable of phosphorylating several non-particled substrates such as tubulins, sinucleins and phosducins. The affinity shown by GRK2 for its substrates is variable. Thus, the Km for sinucleins and for β-tubulins, depending on the subtypes of both, is around 1-2 µM. In the case of p38, the value of the Km=80 nM (see table of Figure 1, panel E) is closer to that described for other physiological substrates of GRK2 such as phosducin and the protein similar to phosducin (PhD and PhLP, Km between 40-100 nM or the m₂-muscarinic receptors. The stoichiometry values found in at least three quantifications by Cerenkov ranged between 0.4-0.8 mol Pᵢ/mol p38, which shows the existence of a phosphorylation site by GRK2.

### GRK2 and p38 interact, depending on the agonist.

GRK2 interacts with several proteins involved both in signaling and cell traffic. Thus, GRK2 interacts with Gαq, Gβγ, PI3Kα and γ, clathrin, GIT (GRK Interacting protein) and caveolin, in addition to other molecules, the interaction of which causes the modulation of its activity (such as phospholipids, Ca²⁺-calmodulina, kinases, etc.). GRK2 is a modular kinase and its catalytic activity is restrained by intramolecular reactions between its different domains. Due to this, in order to observe its ability to associate with p38, the acquisition of its active conformation was provoked by means of stimulation with β₂AR receptors, the activation of p38 by these being scarce. HEK293 cells, transiently transfected with the plasmids encoding the β₂AR receptor, Flag-p38 and GRK2, were used. After subjecting the cells to a nocturnal starvation, they were stimulated with the isoproterenol agonist, at a concentration of 10 µM. In these conditions, the immunoprecipitation brings a detection of the GRK2 kinase in the immunoprecipitates and this effect is increased by stimulation with the β₂AR receptor (Figure 2, panel A). As shown in the different sections forming Figure 2, the maximum association is obtained five minutes after exposure to isoproterenol. On the other hand, it is observed that the association between both kinases is not compulsory of the stimulation of β₂AR, given that they also coimmunoprecipitate in basal conditions (0 minutes). In panel A, the unspecific drag controls (Cneg) of the anti-Flag immunoprecipitation and the verification that another notable stimulation of p38 (NaCl) does not trigger this association are included. Finally, levels of p38 activation in these conditions are shown. These show firstly that p38 activation by isoproterenol is not very strong and secondly; that the activation is smaller the more it bonds to GRK2.

"Reciprocal" immunoprecipitation assays, i.e., in the same cell system but using anti-GRK2 antibodies to detect p38 in the immunoprecipitates, were carried out. In section B, it was observed that p38 and GRK2 interact depending on the agonist (5 minutes of isoproterenol). It was verified, section **C**, that by decreasing the concentration of GRK2, it was still rescued bound to Flag-p38. An increased binding was again found 5 minutes after stimulation by the β₂AR agonist.

In section D, the ability of p38 of coimmunoprecipitating with the catalytically inactive mutant of GRK2, K220R was examined. It can be observed that, at similar expression levels of GRK2-K220R and of GRK2-WT (see third panel), the mutant is not only capable of associating itself to p38 in a greater extent but also its interaction seems to be independent of the β₂AR stimulus.

### The overexpression of GRK2 reduces the ability of p38 of being activated by the MKK6 kinase.

The interphase between p38 and one of its commonest activators, MKK6, was tested. With the purpose of limiting and severing possible crossed activations, the constitutively active mutant of this MAPKK, MKK6CAM was used. Figure 3, section A includes the representative panels of transient transfection experiments in HEK293 cell , in which increasing doses of pCDNA3-GRK2 plasmid were overexpressed (always completing the total amount of DNA with pCDNA3-GFP), together with Flag-p38 and MKK6CAM. It is observed that the p38 activation dependent on MKK6CAM decreased more as more amounts of GRK2 were expressed in the cells. The overexpression allows making the evaluation of the result of the same cells certain. Certainly, since only the immunodetection of Flag-p38 is taken, the transfected cells are selected (and therefore subjected to the effects of GRK2 and MKK6CAM) and the effects of the rest are ignored. On the other hand, the endogenous p38 was difficult to detect in these cells in every occasion. Cells expressing fixed amounts of GRK2, two HEK293 populations stably expressing different amounts of GRK2 were used, the DNAs encoding Flag-38 and MKK6CAM (or its control) were transiently introduced into them. The results are gathered in section B and they show that, in this system, a decrease in the ability of p38 of being activated by MKK6CAM is again observed.

### The overexpression of GRK2 reduces the kinase activity of p38 on a peptide-substrate.

Subsequently, the catalytic activity of a p38 subjected to both the stimulus of its activator MKK6CAM and to the stimulus of increasing doses of GRK2 on an exogenous substrate was tested. For this reason, HEK293 cells were left in medium with serum for the days required for the optimum expression to take place; as a consequence of which GRK2 could have been stimulated by factors present in the serum, such as LPA etc., the signals of which arise from the GPCRs. The cells were collected and the Flag-p38 kinase recognized by the monoclonal anti-flag antibody bound to an agarose resin was immunoprecipitated from them. In each point (always carried out in duplicate) an aliquot of the immunoprecipitate was reserved for checking the amount of the same by immunodetection, (see panel inserted in section B of Figure 4). The immunoprecipitates, washed repeatedly were then tested in a phosphorylation reaction on the peptide substrate APR. This peptide, thus called due to the first three amino acids of its sequence, corresponds to residues 94 to 102 of MBP and has been previously used to test the activity of MAPK for having the phosphorylation consensus of these enzymes. Section of **A** includes lysate controls indicating both the expression level of GRK2 and the activation state that p38 reached in those conditions. The analysis of section B allows issuing different conclusions. The first is that the catalytic activity of immunoprecipitated p38 is correlated, in each point, with its respective activation state, detected with the anti-phospho p38 antibody in **A** and it is characterized by being dimmed by the overexpression of GRK2. The second is that Flag-p38 subjected to stimuli preset in serum of the culture medium also has a catalytic activity that is insignificant compared to the peptide-substrate APR. Therefore, these levels were taken as a reference for the graphic quantification. It has to be emphasized finally that the phosphorylation of the APR peptide was strictly dependent on p38, given its obvious inhibition by SB 203580.

### The reduction of GRK2 levels affects the greater activation of p38 by MKK6_{CAM}.

With the purpose of assuring the previous results, the reciprocal experiments were undertaken. In other words, whether GRK2 is negatively affecting p38 activity. Independently of its kinase activity on GPCRs, the decrease in the GRK2 levels should allow a greater anti-phospho-p38 signal in the same context, by MKK6_{CAM}. HEK293 cells were used for carrying out transient transfections of increasing amounts of DNA antisense (AS) against GRK2 (Figure 5 A, left part) the parallel control of which are the same amounts of plasmid encoding GFP (C). The Figure shows the panels of a representative experiment. In the GRK2 levels, a 20-50 % decrease is obtained on average, according to the DNA dose used. These data corroborate what is inferred from the earlier GRK2 overexpression experiments, namely that the smaller amount of GRK2 in the cells, p38 is capable of suffering a greater activation by MKK6_{CAM}. A graphic estimation representative of this effect is attached (Figure 5 A, right part). The levels of activation of p38 (anti-phospho-p38/anti-p38) refer to the baseline activation in each situation: either greater reduction of GRK2 (AS) or of GFP (C, control).

In these same experiments, the activation of Flag-p38 is assessed in baseline conditions. The resting activity, that is, without MKK6_{CAM}, of p38α is usually scarce. It can be detected by immunodetection by leaving long exposures during chemiluminescent developing (referred to as "overexposure" in Figure 5, section B).Thus, as can be observed, the profile of activation of p38, in conditions of non-co-transfection of MKK6_{CAM}, mimics the profile of p38 subjected to the activation of this constitutively active mutant. According to the elimination of one of the variables, the overexpression of MKK6_{CAM} having a bearing on an inconstant activation of p38, the data of baseline activity of p38 affected b the presence of greater or smaller levels of total GRK2 are more quantitative. Thus, the data from the quantification of the activation of Flag-p38α without MKK6_{CAM} are represented in the lower part of the Figure. Relating the activation of p38 to the parallel control of GFP in each point of DNA antisense, a marked tendency is obtained of p38 being the best substrate of its cell activators the smaller the presence of GRK2 (Figure 5, graph of section B). The significance, denoted by means of an asterisk, is therefore referred to the fact that a value of p<0.05 was obtained in the statistical t-Student test in each point of DNA antisense compared to its respective GFP control.

### The location of the phosphorylation site by GRK2 in p38 by means of truncated construct shows the involvement of tertiary structural determinants in the p38-GRK2 interaction.

Mxi2 is an alternative processing variant of p38 the C-terminal of which differs from that of p38α. Mxi2 is a protein that is initially isolated in double hybrid experiments for interacting with the protein Max. From amino acids 1 to 280, it is identical to p38α but it has a C-terminal of 17 completely different residues (see schemes in Figure 6). In addition to Mxi2, truncated mutant MxiΔ17 has been used (corresponding exactly to a p38αΔ80) to determine of any serine or threonine of the C-terminal of p38 was the target of phosphorylation of GRK2. Fusion proteins were obtained by standard methods pf protein purification and they were tested, their ability of being a GRK2 substrate being compared to p38α (Figure 6, section A). With comparable amounts of p38α, Mxi2 and MxiΔ17, the last two were not phosphorylated by GRK2. Furthermore, phosphorylation traces that can be distinguished in Mxi2 and MxiΔ17 are fainter than that corresponding to phosphorylation of p38α in the presence of heparin. The Figure also includes autophosphorylation controls of each of these kinases.

In view of these results, the generation and purification of the 80 last amino acids of the p38α sequence, fused again to GST. This construct was made by the mutagenesis protocol of QuickChange, and later by later including the DNA fragment encoding the GST-280-360 p38α in el polyvalent system of Gateway. Surprisingly, after purifying the fusion protein and testing it against GRK2, no trace whatsoever of phosphorylation was obtained. These results are shown in section B of Figure 6. These show that with a greater amount of GST-280-360 p38α than of GST-MxiΔ17 and of GST-p38α (WB anti-GST, lower panel), phosphorylation of any of the first two is not obtained and is obtained with the last.

It is inferred from this data that structural determinants present in the protein p38αWT, and absent in its truncated N- and C-terminal parts are needed for the recognition and later phosphorylation by GRK2.

### GRK2 phosphorylates p38 in a single residue.

After verifying the inadequacy of the foregoing approaches for determining the phosphorylated residue by GRK2, the residue was searched for by proteomic techniques, to make certain that the calculated stoichiometry corresponds to the only phosphorylation site, the phosphorylation assays of GRK2 and p38 were resolved by two-dimensional electrophoreses, the results of which is shown in section A of Figure 7. In the first electrophoresis, the change in the isoelectric point of the proteins as a consequence of incorporating the phosphoryl is taken advantage of, while in the second, the proteins are resolved according to their mass, according to a routine SDS-PAGE. Only in the panel corresponding to the phosphorylation in the presence of GRK2 can an intense radioactive band corresponding to GST-p38 be observed. Likewise, in this panel, diffused traces of ³²P can be distinguished, indicating the multiple autophosphorylation of GRK2.

In the Servicio de Proteómica del Nodo UAM of the Cardiovascular Network, included within the Servicio de Proteómica of the Centro de Biología Molecular Severo Ochoa(http://www.cbm.uam.is/mk factory.esdomain/webs/CBMSO/plt_Servicio_Pagina.as px?IdServicio=29&IdObjeto=118), the post-translational modification was identified. By comparing the fragmentation spectra of GST-p38 and of GST-p38 phosphorylated by GRK2, after tryptic digestion and MALDI-TOF mass spectrometry, the existence of a peptide was observed in the phosphorylated sample, the mass of which could correspond to that of another peptide, detected in respective samples, plus 80 Da. Effectively, the minor but exclusive presence of the sample subjected to phosphorylation by GRK2, of this peptide post-translationally modified with a phosphate group (1954.330=1874.326+80) is observed. This Figure (section B of Figure 7) includes the amplification of the area of the spectrum in which traces of this putative phosphorylation carrier were detected, the complete Figure being provided in the section of Materials and Methods. The detailed analysis of the mass spectrum allowed identifying a candidate peptide, result of the digestion with trypsin: LTDDHVQFLIYQILR.

To verify this indication, the candidate peptide was separated by HPLC and a finer spectrometric analysis was carried out: ElectroSpray /Mass Spectrometry- Ionic Tramp (IS/MS-IT), isolatedly monitoring the ion corresponding to the peptide found previously. The results show firstly the information from the high pressure liquid chromatography as regards the elution time of the peptide monitored in both samples. It was observed that the peptide from the phosphorylation by GRK2, left earlier, this is originated by the foundation of chromatography, which separates peptides by hydrophobicity, the less polar peptides being more retained.

Secondly, the fragmentation spectra of both peptides and the assignation of series to the peptides obtained are shown. This analysis allowed identifying the threonine of the candidate peptide LTDDHVQFLIYQILR as the phosphorylation carrier. In the Figure 8, the peaks corresponding to the total mass of the peptide in its different species in the respective samples are highlighted in yellow and the 938.3 Da peak corresponding to the differential species between the spectrum of the phosphorylated sample and the non-phosphorylated sample is highlighted in orange.

The final conclusion of the spectrometric and proteomic approaches was that the phosphorylation of GRK2 on p38 is produced in the threonine 123 of the p38α sequence.

### The mutation of the threonine 123 of p38α prevents the GRK2 phosphorylation

With the purpose of verifying the target residue of the phosphorylation by GRK2, two mutants of p38α in the T123 were generated. The mutant p38αT123A represents the p38 form that cannot be phosphorylated by GRK2 while the mutant p38αT123D, due to the negative charge of aspartic acid as well as to the length of the side chain, mimics the constitutively phosphorylated form of the threonine 123. The fusion proteins GST-p38αT123A and GST-p38αT123D were purified and subjected to the phosphorylation by GRK2, always taking the protein p38αWT as a control (Figure 9, section A). At low and equimolar concentrations of substrate with respect to the kinase, it is confirmed that none of the two mutant is a GRK2 substrate, conforming the identity of threonine 123 as the substrate of said phosphorylation. Section **A** of the Figure also includes controls of the total amount of p38 quantified by immunodetection by an anti-p38 antibody. In **B**, a scaled representative scheme of p38α is included (access number to the database Swiss-Prot Q16539, www.expasy.org), in which the large kinase domain, including almost the entire protein, has to be emphasized. A small area, called CD, is located at the end of the protein and it forms the Common Docking domain for both substrates and activators of p38 and it has been observed that it mediates the protein-protein recognitions in the MAPK family. The analysis of the p38 crystal together with peptides from MKK3 and from MEF2Ahas allowed correcting and perfecting the description of this docking groove.

### The threonine 123 is a residue highly conserved between isoforms and between species.

Figure 10 shows two alignments of the sequences of different p38 proteins made by the inventors. In the upper panel, the α isoforms of the p38 (MK14 in the database Swiss-Prot) of different species have been compared : *Cyprinus carpio* (common carp, MK14A_CYPCA: Q90336), *Drosophila melanogaster* (fruit fly, MK14A_DROME: 062618), *Xenopus laevis* (African clawed frog, MK14_XENLA: P47812), *Pan troglodytes* (chimpanzee, MK14-PANTR: Q95NE7), *Canis familiaris* (dog, MK14_CANFA: 002812), *Homo sapiens* (human, MK14_HUMAN: Q16539), *Mus musculus* (mouse, MK14_MOUSE: P47811) and *Rattus norvegicus* (rat, MK14_RAT: P70618). A simple color code is used to differentiate the identical amino acids between p38 (yellow) orthologues of very conserved amino acids identical to the consensus (blue), of very conserved amino acids equivalent to the consensus (green). The remaining non-conserved residues remain in white. The regions in which a greater variability has been allowed are precisely the ones outside the kinase domain of p38, among which the C-terminal area is emphasized, in which the CD domain is located; the acidic amino acids of this domain allow, thanks to the establishment of electrostatic interactions with basic amino acids of substrates, activators and deactivators, the recognition thereof. The location of the T123 in the sequence of all the aligned p38α is highlighted by means of a red ellipse. Not only is the threonine 123 present in almost all the isoforms but when it does not appear, a serine is found which can be equally phosphorylated in its place. Furthermore, the region lined by the acidic amino acids which is presumed to form the consensus of phosphorylation by GRK2 is conserved in all the orthologues.

Subsequently, it was studied whether the regulation described by the inventors could be common to all four preeminent isoforms of p38, α, β, γ, and δ. For this, the areas comprising the residues 120 to 140 of the p38 isoforms of yeasts: HOG1 of *Candida albicans* (Q92207), HOG1 of *Saccharomyces cerevisiae* (P32485), Styl of *Schizosaccharomyces pombe* (Q09892); the γ isoforms of p38 of human, of mouse, of rat and of African frog (isoforms MK12 in the alignment, P53778, 008911, Q63538, P47812); δ isoforms of p38 of human, of chimpanzee, of mouse and of rat (isoforms MK13: 015264, Q9N272, Q9Z1B7 and Q9WTY9 respectively in the alignment); the two isoforms of p38 of drosophila (MK14_DROME, O62618 and 061443), β isoforms of human (β₂ differs from the initially isolated β in that it lacks the insertion by alternative processing of eight amino acids present in the latter. The β isoform is a minority and is difficult to isolate therefore, the β form is normally identified with the β₂ variant) and of mouse (MK11: Q15759, Q9WUI1); and finally the a isoforms (MK14) of *Xenopus,* of *Cyprinus* (isoform MK14A previously included in the comparison and isoform MK14B, Q91958) of chimpanzee, of dog, of man, of mouse and of rat, previously aligned. The high conservation between all of them, even the ones from yeasts, is striking. With respect to the threonine in question (T123 of p38α human), it can be said that it is found conserved in vertebrates-even in metazoa- as well as the surrounding amino acids, in which the acidic residues D and E are found. Thus, even if the alignment undergoes an interruption at the precise position of this residue, the δ isoforms of p38 are still conserved. The only isoform included in this alignment that lacks a serine or threonine in a homologous context is the human p38γ.

### The phosphorylation of p38 by GRK2 reduces both the catalytic activity of p38 on its substrates and the ability of being a MKK6 substrate.

Figures 11 and 12 show the experiments planned to determine the functional consequences that the phosphorylation of GRK2 could have on the T123 of p38. First, it was studied whether the phosphorylation of GRK2 on p38 would reduce the activation of the latter by MKK6_{CAM} *in vitro.* The results, in duplicate, obtained in the experiments of phosphorylation of MKK6_{CAM} onGST-p38 and the mutants GST-p38T123A and GST-p38T123D are shown I the panels forming A in Figure 11. At similar amounts of recombinant proteins (Coomassie Blue), GST-p38T123D is a much worse MKK6_{CAM} substrate than the other two p38 (³²P).

Subsequently, it was studied whether this decreased ability of GST-p38T123D if phosphorylated by MKK6_{CAM} was correlated to a reduction of its catalytic activity *in vitro.* For this, several phosphorylation assays were made which, due to the undetectable baseline activity of GST-p38, required the inclusion of the activating kinase MKK6_{CAM}. In part B of Figure 11, the ability of GST-p38WT and of the two mutants GST-p38T123A and GST-p38T123D to phosphorylate a known p38 substrate such as ATF2, previously purified as protein of fusion to GST, is tested. Thus, in each phosphorylation reaction, substrate, MAPK and MAPKK are included. A representative experiment (³²P) is shown wherein the points are in duplicate (Coomassie Blue). Thus, these experiments show that p38Tα123D lacks the most minimum catalytic activity on GST-ATF2 while p38α and p38Tα123A phosphorylate it analogously.

It was decide to verify the data with a more specific p38 substrate, since ATF2 is also phosphorylated by JNK. GST-MEF2A was purified, due to the fat that it was recently crystallized together with p38 as well as to the fact that it is a preferred p38 substrate. Figure 12 shows the assays carried out with this substrate. Section A consolidates the previous deductions, the activity of p38Tα123D on MEF2A is completely abolished, while the mutant p38Tα123A conserves the ability to phosphorylate this substrate. It is also observed that the phosphorylation is accompanied by a correlative change in the mobility of the GST-MEF2A substrate, detected by Coomassie Blue staining. It is normal for the hyperphosphorylation of a protein to have a bearing on the change in its electrophoretic mobility, which can be observed even in denaturing polyacrylamide and SDS gels. After analyzing the phosphorylation assays as a whole, it could be further observed that the mutant p38Tα123A, although it is correctly phosphorylated by MKK6_{CAM}, has a reduced kinase activity against the two substrates that were tested. It was decide to dissect this differential effect between p38Tα123A and p38αWT slightly more, for which the inventors focused their attention on their respective baseline activity, i.e. in the absence of the activating kinase MKK6 and against MEF2A (Figure 12, section B). The baseline activity of p38 could be evaluated in conditions of sufficient substrate and long exposure and it could be checked whether respective mutants in the T123 have a smaller baseline catalytic activity than the wild kinase. p38Tα123A behaved like the middle ground between p38αWT and p38Tα123D. Therefore, the mutant p38Tα123A, in more restrictive enzymatic conditions (10 times les substrate and 15 minutes of phosphorylation; panel C of Figure 12), has a grater catalytic difference with respect to a p38αWT than the initial experiments showed.

### The p38T123D mutant has lower ability of being activated by MKK6_{CAM} in situ.

The results, which parallelly provide an explanation consistent with the initial data in HEK293 cells, were corroborated in this same system. First, the expression pCDNA3-Flag-p38T123D mutant was generated by PCR in eukaryotes. Then, following experimental approaches analogous to overexpression or reduction of GRK2 in HEK293, Flag-p38WT and Flag-p38T123D were transfected together with the activator MKK6_{CAM}. The graph in Figure 13 shows, by comparing the activation of Flag-p38T123D (versus its baseline activation) with the activation of Flag-p38WT, a considerable reduction thereof. The activation of p38T123D in cells is less likely that that of wild kinase. Illustrative panels of the immunodetections provided by these experiments are also included in this Figure.

In figure 14, the binding of substrates (MK2) and activators (MKK6) to p38 or its mutants in T123 is analyzed by an in vitro binding assay using purfied proteins. As is shown, the ability of the T123D mutant to associate to MK2 or MKK6 is severely impaired with respect to the T123A mutant or the wild type protein.

### GRK2 negatively regulates differentiation of the preadipocytic 3T3- L1 line induced by insulin.

The preadipocytic line 3T3- L1 was used as a cell model that allowed studying the regulation of p38 by GRK2. These fibroblasts have the interesting particularity that when subjected to certain stimuli, among which insulin stands out, they acquire an adipocytic phenotype at the end of an approximately two-week treatment. This differentiation can be easily distinguished by the accumulation of drops of fat that are stained with a red lipophilic coloring and which occupy almost the entirety of the cytoplasm of the 3T3-L1.

One of the physiological functions of p38, other than the most orthodox response to cell stress, is its role in the differentiation process. In the specific case of 3T3L1 fibroblasts, the involvement of p38 has been demonstrated, in so far as the differentiation into adipocytes is blocked by SB203580 and the presence of MKK6_{CAM} is sufficient. The transcription factors C/EBP (CCAAT/enhancer-binding protein) and PPARγ (peroxisome proliferator-activated receptor γ), the expression of which changes over the course of differentiation, seem subjected to regulation by p38. More specifically, C/EBP β is supposedly phosphorylated by p38, which in turn is active only in the initial steps of differentiation, which would promote the later expression of PPARγ and of the adipocytic markers that are under its control.

To investigate the effect that GRK2 may have on adipocytic differentiation of 3T3L1, lines were generated that were stably transfected with plasmids pCDNA3-GRK2 and pCDNA3-GRK2K220R providing resistance to neomycin. After the relevant verification that the GRK2 levels were effectively overexpressed in both cases in comparison to line 3T3L1 (window inserted in the graph of Figure 15), differentiation was triggered according to the standard protocol. The 3T3L1 cells, in the absence of lipogenic stimuli, have a healthy appearance of fibroblasts (photo corresponding to the control in 3T3L1) clearly converted into an adipocytic phenotype as shown by the red lipidic drops and the rounded morphology these cells acquire (photo + insulin in 3T3L1). In the case of stable GRK2, the number of adipocytes per field is visibly less that in the case of the fibroblasts with endogenous levels of this kinase. And as regards the 3T3L1-K220R cells, the adipogenic effect is substantially greater than that of the control cells. GRK2 therefore inhibits the acquisition of the adipocytic morphology mediated by insulin while K220R stimulates it. Furthermore, this effect is dependent on p38 as the addition of the pharmacological inhibitor SB203580 reverses the entire adipogenesis. Furthermore, when plates not subjected to the differentiating treatments were reserved as internal controls for the experiment, the stable K220R will experience much greater spontaneous conversion into adipocytes than the other cells. The count of the number of adipocytes per field in each one of the three cell lines is represented in the graph on the right-hand side. Field is understood to be an area viewed with the optical microscope in each p100 or p60. Normally up to a total of 25 random fields were counted in each plate. The significance of the data was calculated by means of the Student's t-test (p<0.001) for paired data (each stable cell line compared with 3T3L1).

Data from figure 16 and 17 lead to the conclusion that a polyclonal antiserum raised against a peptide of p38 phosphorylated in T123 is able to recognize the p38 protein in vitro phosphorylated by GRK2 but not by MKK6 in other residues, and that this recognition is specific for the T123 residue since the T123A mutant is not immunodetected by this antibody. Also that overespression of GRK2 can promote an increase in the immunodetection of this epitope by this antibody, signal that is decreased when a GRK2 inactive mutant (K220R) is overexpressed.

**TABLE I**

| **Antibody (Ac)** | **Epitope/ Protein Mw(KDa)** | **Type** | **Dilution (WB)** | **Dilution (IP or IF)** | **Immunogenic peptide** | **Company/ Source** |
|---|---|---|---|---|---|---|
| Anti-HA (12CA5) | HA 1.1 | MR | - | 2µg/ml | Residues 76-111 of hemagglutinin : nonapeptide YPYDVPDYA | Boehringer Mannheim |
| Anti-HA (Y-11) | HA | PC | 1:500 | - | Internal residues of hemagglutinin | Santa Cruz |
| Anti-Flag M2-agarose | FLAG 0.96 | MR bound to agarose resin | - | 0.01-0.02 µl of Ac-resin/µl of lysis buffer | FLAG octa-peptide (DYKDDDDK) | Sigma |
| Anti-Myc-agarose | c-Myc 2.1 | MR bound to agarose resin | - | 0.01-0.02 µl of AC-resin/µl of lysis buffer | Peptide corresponding to amino acids 408-439 of human c-Myc MEQKLISEEDLLRKRGST | Santa Cruz |
| Anti-Myc | c-Myc 2.1 | MR | - | 1:1000 (If) | Hybridoma 9E10 against human sequence EQKLISEEDL | Servicio de Microscopía (Microscopy Service) of CBMSO |
| Anti-PF1(GRK2) | GRK2 79.6 | PC | 1:1000 | 1:300 | Fusion protein GST-PF1 (amino acids 50-145 of bovine GRK2) | Own production (Dr. C. Murga) |
| 910 (GRK2) | GRK2 | PC | 1:1000 | - | Fusion protein GST-PF1 (amino acids 50-145 of bovine GRK2). | Own production (Dr. E. Morato and B. Palacios) |
| Anti-PF2 (GRK2) | GRK2 | PC | 1:1000 | 1:200 | Fusion protein GST-PF2 (amino acids 436-689 of bovine GRK2) | Own production (Dr. C. Murga) |
| Anti-GRK2/3 | GRK2/3 | MR | 1:1000 | 1 µg/ml | Fusion proteinGST-GRK3 (467-688). | Upstate Biotechnology |
| Anti-GRK3 (sc-563) | GRK3 79.7 | PC | 1:1000 | - | C-terminal peptide of bovine GRK3 | Santa Cruz Biotechnology Santa Cruz Biotechnology |
| Anti-GRK5 (sc-565) | GRK5 67.8 | PC | 1:1000 | 0.2 µg/ml | C-terminal peptide of human GRK5 | Santa Cruz Biotechnology |
| Anti-GRK6 (sc-566) | GRK6 66 | PC | 1:500 1 | 0.2 µg/ml | C-terminal peptide of human GRK6 | Santa Cruz Biotechnology |
| Anti-ERK1 (sc-93) | ERK1 P44 | PC | 0.75 µg/ml | - | C-terminal replaced peptide of rat ERK1 | Santa Cruz Biotechnology |
| Anti-ERK2 (sc-154) | ERK2 42 | PC | 0.75 µg/ml | - | C-terminal replaced peptide of rat ERK2 | Santa Cruz Biotechnology |
| Anti-P-ERK 1/2 | ERK1 and ERK2 | MR | 1:500 | - | Phospho T202/Y204. (active form). Synthetic peptide corresponding to the region around said residues of human p44MAPK | Cell Signaling |
| Anti-p38 | p38 42 | PC | 1:1000 | - | Synthetic peptide derived from human p38 | Cell Signaling |
| Anti-p38N | P38 | PC | 1:1500 | - | Last 14 amino acids (C-terminal) of Xenopus p38α terminal) of Xenopus p38α | Donated by Dr. A. Nebreda (EMBL, Heidelberg, Germany. CNIO, Madrid, Spain) |
| Anti-P-p38 | p38 | PC | 1:1000 | - | Phospho T180/Y182. Synthetic peptide corresponding to the region around said residues of human p38. | Cell Signaling |
| Anti-MKK6/ SKK3 | MKK6 40 | PC | 1:500 | - | Human Fusion protein MBP- MKK6 | Upstate Biotechnology |
| Anti-MKK6/ SKK3 | MKK6 | PC | 1:1000 | - | Human fusion protein MalE- MKK6 | Donated by Dr. A. Nebreda (EMBL, Heidelberg, Germany. CNIO, Madrid, Spain) |
| Anti-Gαs (K-20) | Gαs 45 | PC | 1:1000 | - | N-terminal epitope of human Gαs | Santa Cruz |
| Anti-Gαq/1.1(C-19) | Gαq/11 42 | PC | 1:1000 | - | Peptide within domain common to mouse Gαq and Gαl1. | Santa Cruz Santa Cruz |
| Common anti-Gαl | Gαl-1 (I-20), Gαl-2(T-19) and Gαi-3(C-10) 41-45 | PC | 1:1000 | - | Own mixture of antibodies against the different human and rat Gαi. | Santa Cruz |
| Anti-Gα12 (S-20) | Gα12 44 | PC | 1:1000 | - | N-terminal peptide of mouse Gα12 | Santa Cruz |

## Claims

1. An MAPK protein, wherein said MAPK protein is a p38 protein, selected from:
a) an MAPK protein comprising a phosphorylated residue in a phosphorylation site that is different from the phosphorylation site or sites present in the activation segment of said MAPK protein, or a fragment of said protein comprising said phosphorylated residue, wherein
- said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
- the phosphorylation at said different phosphorylation site prevents the activation of said MAPK protein and also its activity towards its substrates; and
b) an MAPK protein comprising a negative charge or a bulky residue in a phosphorylation site, or at the area surrounding said phosphorylation site, that is different from the phosphorylation site or sites present in the activation segment of said MAPK protein, or a fragment of said protein comprising said phosphorylated residue, wherein
- said different phosphorylation site is the threonine residue in position 123 (Thr123) of mouse p38, α isoform, or a residue of a positionally equivalent amino acid susceptible of phosphorylation in another MAPK protein as it is defined by multiple alignment of amino acid sequences, and
- the introduction of a negative charge or a bulky residue at said phosphorylation site, or at the area surrounding said phosphorylation site, prevents the activation of said MAPK protein and also its activity towards its substrates.

2. Protein according to claim 1, wherein said MAPK protein is mammal p38.

3. Protein according to claim 1, wherein said MAPK protein is mouse p38, α isoform and has the amino acid sequence shown in SEQ ID NO: 1.

4. Protein according to claim 1, wherein said MAPK protein comprises an activation segment selected from:
- an activation segment comprising the amino acid triad of formula (I)
Thr-Xaa-Tyr (I)
where
Thr is threonine,
Tyr is tyrosine, and
Xaa is the residue of an amino acid, preferably, of an amino acid selected from aspartic acid, glutamic acid, glutamine, glycine and proline; and
- an activation segment comprising the amino acid triad of formula (II)
Ser-Glu-Gly (II)
where
Ser is serine,
Glu is glutamic acid, and
Gly is glycine.

5. A protein according to claim 1, comprising the amino acid sequence shown in SEQ ID NO: 2.

6. An *in vitro* method for identifying a potentially useful compound for the treatment of pathologies mediated by active MAPK proteins, wherein said MAPK protein is a p38 protein, comprising:
a) placing the candidate compound in contact with said MAPK protein,
b) detecting the phosphorylation of said MAPK protein in a phosphorylation site different from the phosphorylation site or sites present in the activation segment of said MAPK protein, and
c) analyzing if said phosphorylation site (i) is Thr123 of mouse p38, α isoform, in the event that the MAPK protein used was said protein, or a residue of a positionally equivalent amino acid susceptible of phosphorylation in another MAPK protein as it is defined by multiple alignment of amino acid sequences, and if (ii) the phosphorylation in said different phosphorylation site prevents the activation of said MAPK protein;
or alternatively,
i) placing a candidate compound capable of phosphorylating said MAPK protein in contact with an MAPK protein;
ii) detecting the phosphorylation of said MAPK protein in a phosphorylation site of the activation segment of said MAPK protein to measure the effect of the candidate compound on the activation of the MAPK;
iii) analyzing the activity of the said MAPK protein in the presence of the candidate compound towards its substrates to test the possible inhibition of the docking and/or activity of the MAPK protein to its substrates in the presence of a competing compound; and
iv) analyzing if said phosphorylation site (i) at Thr123 of mouse p38, α isoform, in the event that the MAPK protein used was said protein, or a residue of a positionally equivalent amino acid susceptible of phosphorylation in another MAPK protein as it is defined by multiple alignment of amino acid sequences, and if (ii) the phosphorylation in said phosphorylation site prevents the activation of said MAPK protein.

7. An antibody able to bind to an MAPK protein according to any of claims 1 to 5, and/or able to detect said MAPK protein according to any of claims 1 to 5, wherein said antibody is able to specifically recognize the phosphorylated Thr123_amino acid residue or an epitope of said MAPK protein comprising the phosphorylated Thr123.

8. Antibody according to claim 7, wherein said antibody is able to bind to an epitope comprised in a fragment of the mammal p38 kinase, said fragment comprising a phosphorylated Thr123 residue, or a positionally equivalent residue in other p38 MAPK proteins.

9. Antibody according to claim 7 or 8, wherein said antibody is able to bind to an epitope comprising the amino acid sequence shown in SEQ ID NO: 2.

10. A pharmaceutical composition comprising a therapeutically effective amount of an antibody according to any of claims 7 to 9, together with, optionally, a pharmaceutically acceptable carrier.

11. The use of:
(i) an antibody according to any of claims 7 to 9; or
(ii) a GRK2 protein; or
(iii) a vector comprising a nucleic acid encoding GRK2;
in the manufacture of a pharmaceutical composition for the treatment of a pathology mediated by active MAPKs, wherein said MAPK protein is a mammal p38 protein and the pathology is aortic constriction, heart failure following advanced coronary disease, congestive heart failure or rheumatoid arthritis.

12. A kit comprising an MAPK protein according to any of claims 1 to 5, or an antibody according to any of claims 7 to 9.

13. Kit according to claim 12, wherein said MAPK protein is a phosphorylated mammal p38 kinase in Thr123 of mammal p38, α isoform.

## Patentansprüche

1. MAPK-Protein, wobei das MAPK-Protein ein p38-Protein ist, ausgewählt aus:
a) einem MAPK-Protein, umfassend einen phosphorylierten Rest an einer Phosphorylierungsstelle, die unterschiedlich zu der/den Phosphorylierungsstelle oder -stellen in dem Aktivierungssegment des MAPK-Proteins ist, oder einem Fragment des Proteins, welches den phosphorylierten Rest umfasst, wobei
- die unterschiedliche Phosphorylierungsstelle der Threoninrest an Position 123 (Thr123) der α-Isoform von Maus-p38 ist, oder ein Rest einer positionell gleichwertigen Aminosäure, die anfällig für Phosphorylierung ist, in einem anderen MAPK-Protein wie es durch mehrere Aminosäuresequenzabgleiche definiert ist, und
- die Phosphorylierung an der unterschiedlichen Phosphorylierungsstelle die Aktivierung des MAKP-Proteins sowie auch seine Aktivität gegenüber seinen Substraten verhindert; und
b) einem MAPK-Protein, umfassend eine negative Ladung oder einen sperrigen Rest in einer Phosphorylierungsstelle, oder in dem Bereich, welcher die Phosphorylierungsstelle umgibt, die unterschiedlich zu der/den Phosphorylierungsstelle oder -stellen in dem Aktivierungssegment des MAPK-Proteins ist, oder einem Fragment des Proteins, welches den phosphorylierten Rest umfasst, wobei
- die unterschiedliche Phosphorylierungsstelle der Threoninrest an Position 123 (Thr123) der α-Isoform von Maus-p38 ist, oder ein Rest einer positionell gleichwertigen Aminosäure, die anfällig für Phosphorylierung ist, in einem anderen MAPK-Protein wie es durch mehrere Aminosäuresequenzabgleiche definiert ist, und
- die Einführung einer negativen Ladung oder eines sperrigen Rests an der Phosphorylierungsstelle, oder an dem die Phosphorylierungsstelle umgebenden Gebiet die Aktivierung des MAPK-Proteins und auch seine Aktivität gegenüber seinen Substraten verhindert.

2. Protein gemäß Anspruch 1, wobei das MAPK-Protein Säuger-p38 ist.

3. Protein gemäß Anspruch 1, wobei das MAPK-Protein die α-Isoform von Maus-p38 ist und die Aminosäuresequenz wie in SEQ ID NO: 1 gezeigt ist.

4. Protein gemäß Anspruch 1 , wobei das MAPK-Protein ein Aktivierungssegment umfasst, welches ausgewählt ist aus:
- einem Aktivierungssegment umfassend die Aminosäure-Dreiergruppe der Formel (I)
Thr-Xaa-Tyr (I)
wo
Thr Threonin ist,
Tyr Tyrosin ist, und
Xaa ein Rest einer Aminosäure ist, vorzugsweise einer Aminosäure ausgewählt aus Asparaginsäure, Glutaminsäure, Glutamin, Glycin und Prolin; und
- einem Aktivierungssegment, umfassend die Aminosäure-Dreiergruppe der Formel (II)
Ser-Glu-Gly (II)
wo
Ser Serin ist,
Glu Glutaminsäure ist, und
Gly Glycin ist.

5. Protein gemäß Anspruch 1, umfassend die Aminosäuresequenz wie in SEQ ID NO: 2 gezeigt ist.

6. *In vitro*-Verfahren zur Identifizierung einer potentiell nützlichen Verbindung zur Behandlung von Pathologien, die durch aktive MAPK-Proteine vermittelt werden, wobei das MAPK-Protein ein p38-Protein ist, umfassend:
a) Inkontaktbringen der Kandidatenverbindung mit dem MAPK-Protein,
b) Nachweis der Phosphorylierung des MAPK-Proteins an einer Phosphorylierungsstelle, die unterschiedlich zu der/den Phosphorylierungsstelle oder -stellen in dem Aktivierungssegment des MAPK-Proteins ist; und
c) Untersuchung, ob die Phosphorylierungsstelle (i) Thr123 der α-Isoform von Maus-p38 ist, unter der Annahme, dass das verwendete MAPK-Protein das Protein war, oder ein Rest einer positionell gleichwertigen Aminosäure, die anfällig für Phosphorylierung ist, in einem anderen MAPK-Protein wie es durch mehrere Aminosäuresequenzabgleiche definiert ist, und ob (ii) die Phosphorylierung an der unterschiedlichen Phosphorylierungsstelle die Aktivierung des MAPK-Proteins verhindert;
oder alternativ
i) Inkontaktbringen einer Kandidatenverbindung, welche fähig ist, das MAPK-Protein zu phosphorylieren, mit einem MAPK-Protein;
ii) Nachweis der Phosphorylierung des MAPK-Proteins an einer Phosphorylierungsstelle des Aktivierungssegments des MAPK-Proteins, um den Effekt der Aktivierung des MAPK durch die Kandidatenverbindung zu messen;
iii) Untersuchung der Aktivität des MAPK-Proteins in Gegenwart der Kandidatenverbindung gegenüber seinen Substraten, um die mögliche Inhibierung der Kopplung und/oder Aktivität des MAPK-Proteins zu seinen Substraten in der Gegenwart einer konkurrierenden Verbindung zu testen; und
iv) Untersuchung, ob die Phosphorylierungsstelle (i) an Thr123 der α-Isoform von Maus-p38, unter der Annahme, dass das verwendete MAPK-Protein das Protein war, oder ein Rest von einer positionell gleichwertigen Aminosäure, die anfällig für Phosphorylierung ist, in einem anderen MAPK-Protein wie es durch mehrere Aminosäuresequenzabgleiche definiert ist, und ob (ii) die Phosphorylierung an der Phosphorylierungsstelle die Aktivierung des MAPK-Proteins verhindert.

7. Antikörper, welcher fähig ist, an das MAPK-Protein gemäß einem der Ansprüche 1 bis 5 zu binden, und/oder fähig ist, das MAPK-Protein gemäß einem der Ansprüche 1 bis 5 nachzuweisen, wobei der Antikörper fähig ist, den phosphorylierten Thr123-Aminosäurerest oder ein Epitop des MAPK-Proteins, welches das phosphorylierte Thr123 umfasst, spezifisch zu erkennen.

8. Antikörper gemäß Anspruch 7, wobei der Antikörper fähig ist, an ein Epitop zu binden, welches in einem Fragment der Säuger-p38-Kinase umfasst ist, wobei das Fragment einen phosphorylierten Thr123-Rest umfasst, oder einen positionell äquivalenten Rest in anderen p38-MAPK-Proteinen.

9. Antikörper gemäß Anspruch 7 oder 8, wobei der Antikörper fähig ist, an ein Epitop, welches die Aminosäuresequenz wie in SEQ ID NO: 2 gezeigt ist, zu binden.

10. Arzneimittel, umfassend eine therapeutisch wirksame Menge eines Antikörpers gemäß einem der Ansprüche 7 bis 9, zusammen mit, gegebenenfalls, einem pharmazeutisch verträglichen Träger.

11. Verwendung von
(i) einem Antikörper gemäß einem der Ansprüche 7 bis 9, oder
(ii) einem GRK2-Protein; oder
(iii) einem Vektor, umfassend eine Nucleinsäure, die GRK2 codiert, in der Herstellung eines Arzneimittels zur Behandlung einer Pathologie, die durch aktive MAPKs vermittelt wird, wobei das MAPK-Protein ein Säuger-p38-Protein ist, und die Pathologie Aortenverengung, Herzinsuffizienz in Folge einer fortgeschrittenen Koronarerkrankung, kongestive Herzinsuffizienz oder rheumatoide Arthritis ist.

12. Kit umfassend ein MAPK-Protein gemäß einem der Ansprüche 1 bis 5 oder einen Antikörper gemäß einem der Ansprüche 7 bis 9.

13. Kit gemäß Anspruch 12, wobei das MAPK-Protein eine an Thr123 der α-Isoform von Säuger-p38 phosphorylierte Säuger-p38-Kinase ist.

## Revendications

1. Protéine MAPK, ladite protéine MAPK étant une protéine p38, choisie entre :
a) une protéine MAPK comprenant un résidu phosphorylé dans un site de phosphorylation qui est différent du ou des sites de phosphorylation présents dans le segment d'activation de ladite protéine MAPK, ou un fragment de ladite protéine comprenant ledit résidu phosphorylé, où
- ledit site de phosphorylation différent est le résidu thréonine en position 123 (Thr123) de l'isoforme α de p38 de souris, ou un résidu d'un aminoacide de position équivalente sensible à la phosphorylation dans une autre protéine MAPK tel qu'il est défini par un alignement multiple de séquences d'aminoacides, et
- la phosphorylation audit site de phosphorylation différent empêche l'activation de ladite protéine MAPK et également son activité vis-à-vis de ses substrats ; et
b) une protéine MAPK comprenant un charge négative ou un résidu volumineux dans un site de phosphorylation, ou bien au niveau de la zone entourant ledit site de phosphorylation, qui est différent du ou des sites de phosphorylation présents dans le segment d'activation de ladite protéine MAPK, ou un fragment de ladite protéine comprenant ledit résidu phosphorylé, où
- ledit site de phosphorylation différent est le résidu thréonine en position 123 (Thr123) de l'isoforme α de p38 de souris, ou un résidu d'un aminoacide de position équivalente sensible à la phosphorylation dans une autre protéine MAPK tel qu'il est défini par un alignement multiple de séquences d'aminoacides, et
- l'introduction d'une charge négative ou d'un résidu volumineux au niveau dudit site de phosphorylation, ou au niveau de la zone entourant le site de phosphorylation, empêche l'activation de ladite protéine MAPK et également son activité vis-à-vis de ses substrats.

2. Protéine suivant la revendication 1, dans laquelle ladite protéine MAPK est la p38 de mammifère.

3. Protéine suivant la revendication 1, dans laquelle ladite protéine MAPK est l'isoforme α de p38 de souris et possède la séquence d'aminoacides représentée dans la SEQ ID N° 1.

4. Protéine suivant la revendication 1, dans laquelle ladite protéine MAPK comprend un segment d'activation choisi entre :
- un segment d'activation comprenant la triade d'aminoacides de formule (I)
Thr-Xaa-Tyr (I)
dans laquelle
Thr représente la thréonine,
Tyr représente la tyrosine, et
Xaa représente le résidu d'un aminoacide, de préférence d'un aminoacide choisi entre l'acide aspartique, l'acide glutamique, la glutamine, la glycine et la proline ; et
- un segment d'activation comprenant la triade d'aminoacides de formule (II)
Ser-Glu-Gly (II)
dans laquelle
Ser représente la sérine,
Glu représente l'acide glutamique, et
Gly représente la glycine.

5. Protéine suivant la revendication 1, comprenant la séquence d'aminoacides représentée dans la SEQ ID N° 2.

6. Méthode *in vitro* pour identifier un composé potentiellement utile pour le traitement de pathologies à médiation par des protéines MAPK actives, dans laquelle ladite protéine MAPK est une protéine p38, consistant à :
a) placer le composé candidat en contact avec ladite protéine MAPK ;
b) détecter la phosphorylation de ladite protéine MAPK dans un site de phosphorylation différent du ou des sites de phosphorylation présents dans le segment d'activation de ladite protéine MAPK, et
c) analyser si ledit site de phosphorylation (i) est Thr123 de l'isoforme α de p38 de souris, dans le cas où la protéine MAPK utilisée était ladite protéine, ou un résidu d'un aminoacide de position équivalente sensible à la phosphorylation dans une autre protéine MAPK tel qu'il est défini par un alignement multiple de séquences d'aminoacides, et si (ii) la phosphorylation dans ledit site de phosphorylation différent empêche l'activation de ladite protéine MAPK ;
ou bien, en variante,
i) placer un composé candidat apte à la phosphorylation de ladite protéine MAPK en contact avec une protéine MAPK ;
ii) détecter la phosphorylation de ladite protéine MAPK dans un site de phosphorylation du segment d'activation de ladite protéine MAPK pour mesurer l'effet du composé candidat sur l'activation de la MAPK ;
iii) analyser l'activité de ladite protéine MAPK en présence du composé candidat vis-à-vis de ses substrats pour tester l'inhibition possible de l'arrimage et/ou de l'activité de la protéine MAPK à ses substrats en présence d'un composé compétitif ; et
iv) analyser si ledit site de phosphorylation (i) est situé à Thr123 de l'isoforme α de p38 de souris, dans le cas où la protéine MAPK utilisée était ladite protéine, ou un résidu d'un aminoacide de position équivalente sensible à la phosphorylation dans une autre protéine MAPK tel qu'il est défini par un alignement multiple de séquences d'aminoacides, et si (ii) la phosphorylation dans ledit site de phosphorylation empêche l'activation de ladite protéine MAPK.

7. Anticorps apte à la liaison à une protéine MAPK suivant l'une quelconque des revendications 1 à 5, et/ou apte à la détection de ladite protéine MAPK suivant l'une quelconque des revendications 1 à 5, ledit anticorps étant capable de reconnaître spécifiquement le résidu d'aminoacide Thr123 phosphorylé ou un épitope de ladite protéine MAPK comprenant le Thr123 phosphorylé.

8. Anticorps suivant la revendication 7, ledit anticorps étant capable de se lier à un épitope présent dans un fragment de la kinase p38 de mammifère, ledit fragment comprenant un résidu Thr123 phosphorylé, ou un résidu de position équivalente dans d'autres protéines MAPK p38.

9. Anticorps suivant la revendication 7 ou 8, ledit anticorps étant capable de se lier à un épitope comprenant la séquence d'aminoacides représentée dans la SEQ ID N° 2.

10. Composition pharmaceutique comprenant une quantité thérapeutiquement efficace d'un anticorps suivant l'une quelconque des revendications 7 à 9, conjointement avec, facultativement, un support pharmaceutiquement acceptable.

11. Utilisation .
(i) d'un anticorps suivant l'une quelconque des revendications 7 à 9 ; ou
(ii) d'une protéine G1RK2 ; ou
(iii) d'un vecteur comprenant un acide nucléique codant pour GRK2
dans la production d'une composition pharmaceutique pour le traitement d'une pathologie à médiation par des MAPK actives, dans laquelle ladite protéine MAPK est une protéine p38 de mammifère et la pathologie est une constriction aortique, une insuffisance cardiaque après une maladie coronarienne avancée, l'insuffisance cardiaque congestive ou la polyarthrite rhumatoïde.

12. Kit comprenant une protéine MAPK suivant l'une quelconque des revendications 1 à 5, ou un anticorps suivant l'une quelconque des revendications 7 à 9.

13. Kit suivant la revendication 12, dans lequel ladite protéine MAPK est une kinase p38 de mammifère phosphorylée dans Thr133 de l'isoforme α de p38 de mammifère.


### SEQUENCE LISTING

<110> universidad Autónoma de Madrid
<120> NEW PHOSPHORYLATION SITE OF MITOGEN-ACTIVATED PROTEIN KINASES, MODIFIED PROTEINS AND APPLICATIONS
<130> P1467PC00
<150> P200501404
   <151> 2005-06-10
<160> 10
<170> PatentIn version 3.3
<210> 1
   <211> 360
   <212> PRT
   <213> Mus musculus
<400> 1
   Met Ser Gln Glu Arg Pro Thr Phe Tyr Arg Gln Glu Leu Asn Lys Thr
   1               5                   10                  15
   Ile Trp Glu Val Pro Glu Arg Tyr Gln Asn Leu Ser Pro Val Gly Ser
               20                  25                  30
   Gly Ala Tyr Gly Ser Val Cys Ala Ala Phe Asp Thr Lys Thr Gly His
           35                  40                  45
   Arg Val Ala Val Lys Lys Leu Ser Arg Pro Phe Gln Ser Ile Ile His
       50                  55                  60
   Ala Lys Arg Thr Tyr Arg Glu Leu Arg Leu Leu Lys His Met Lys His
   65                  70                  75                  80
   Glu Asn Val Ile Gly Leu Leu Asp Val Phe Thr Pro Ala Arg Ser Leu
                   85                  90                  95
   Glu Glu Phe Asn Asp Val Tyr Leu Val Thr His Leu Met Gly Ala Asp
               100                 105                 110
   Leu Asn Asn Ile Val Lys Cys Gln Lys Leu Thr Asp Asp His Val Glu
           115                 120                 125
   Phe Leu Ile Tyr Gln Ile Leu Arg Gln Leu Lys Tyr Ile His Ser Ala
       130                 135                 140
   Asp Ile Ile His Arg Asp Leu Lys Pro Ser Asn Leu Ala Val Asn Glu
   145                 150                 155                 160
   Asp Cys Glu Leu Lys Ile Leu Asp Phe Gly Leu Ala Arg His Thr Asp
                   165                 170                 175
   Asp Glu Met Thr Gly Tyr Val Ala Thr Arg Trp Tyr Arg Ala Pro Glu
   180 185 190
   Ile Met Leu Asn Trp Met His Tyr Asn Gln Thr Val Asp Ile Trp Ser
   195 200 205
   Val Gly Cys Ile Met Ala Glu Leu Leu Thr Gly Arg Thr Leu Phe Pro
   210 215 220
   Gly Thr Asp His Ile Asp Gln Leu Lys Leu Ile Leu Arg Leu Val Gly
   225 230 235 240
   Thr Pro Gly Ala Gln Leu Leu Lys Lys Ile Ser Ser Glu Ser Ala Arg
   245 250 255
   Asn Tyr Ile Gln Ser Leu Ala Glu Met Pro Lys Met Asn Phe Ala Asn
   260 265 270
   Val Phe Ile Gly Ala Asn Pro Leu Ala Val Asp Leu Leu Glu Lys Met
   275 280 285
   Leu Val Leu Asp Ser Asp Lys Arg Ile Thr Ala Ala Gln Ala Leu Ala
   290 295 300
   His Ala Tyr Phe Ala Gln Tyr His Asp Pro Asp Asp Glu Pro Val Ala
   305 310 315 320
   Asp Pro Tyr Asp Gln Ser Phe Glu Ser Arg Asp Leu Leu Ile Asp Glu
   325 330 335
   Trp Lys Ser Leu Thr Tyr Asp Glu Val Ile Ser Phe Val Pro Pro Pro
   340 345 350
   Leu Asp Gln Glu Glu Met Glu Ser
   355 360
<210> 2
   <211> 13
   <212> PRT
   <213> Mus musculus
<400> 2
   Gln Lys Leu Thr Asp Asp His Val Glu Phe Leu Ile Tyr
   1 5 10
<210> 3
   <211> 36
   <212> DNA
   <213> Artificial
<220>
   <223> Forward mutagenic oligonucleotide used for the p38T123A mutant
<400> 3
   gtgaagtgcc agaagctggc cgacgaccac gttcag 36
<210> 4
   <211> 36
   <212> DNA
   <213> Artificial
<220>
   <223> Reverse mutagenic oligonucleotide used for the p38T123A mutant
<400> 4
   ctgaacgtgg tcgtcggcca gcttctggca cttcac 36
<210> 5
   <211> 36
   <212> DNA
   <213> Artificial
<220>
   <223> Forward mutagenic oligonucleotide used for the p38T123D mutant
<400> 5
   gtgaagtgcc agaagctgga cgacgaccac gttcag 36
<210> 6
   <211> 36
   <212> DNA
   <213> Artificial
<220>
   <223> Reverse mutagenic oligonucleotide used for the p38T123D mutant
<400> 6
   gtgaagtgcc agaagctgga cgacgaccac gttcag 36
<210> 7
   <211> 55
   <212> DNA
   <213> Artificial
<220>
   <223> GTW-FWD oligonucleotide used to make the truncated protein GST-280-360p38
<400> 7
   ggggacaagt ttgtacaaaa aagcaggctt cgctgtcgac ctactggaga agatg 55
<210> 8
   <211> 54
   <212> DNA
   <213> Artificial
<220>
   <223> GTW-REV oligonucleotide used to make the truncated protein GST-280-360p38
<400> 8
   ggggaccact ttgtacaaga aagctgggtc tcaggactcc atttcttctt ggtc 54
<210> 9
   <211> 22
   <212> DNA
   <213> Artificial
<220>
   <223> oligonucleotide used for the expression and purification of MAPKAPK2 (MK2)
<400> 9
   ggggccatgg tcaagtccgg cc 22
<210> 10
   <211> 28
   <212> DNA
   <213> Artificial
<220>
   <223> Oligonucleotide used for the expression and purification of MAPKAPK2 (MK2)
<400> 10
   ccccctcgag gtgggccaga gccgcagc 28